Europäisches Patentamt

European Patent Office

Office européen des brevets

(11) Publication number: **0 214 520**
**A2**

(12) **EUROPEAN PATENT APPLICATION**

(21) Application number: **86111527.7**

(22) Date of filing: **20.08.86**

(51) Int. Cl.⁴: **G 01 N 33/574**
**G 01 N 33/577, C 12 N 15/00**
**C 12 P 21/00, C 12 N 5/00**

(30) Priority: **21.08.85 US 767862**
**23.07.86 US 885617**

(43) Date of publication of application:
**18.03.87 Bulletin 87/12**

(84) Designated Contracting States:
**AT BE CH DE FR GB IT LI LU NL SE**

(71) Applicant: **Oncogene Science, Inc.**
**222 Station Plaza North**
**Mineola New York 11501(US)**

(72) Inventor: **Albanese, Joseph M.**
**76 West End Avenue**
**Valley Stream New York 11580(US)**

(72) Inventor: **Reynolds, Frederick H.**
**5 Walker Road**
**Syosset New York 11791(US)**

(72) Inventor: **Stephenson, John R.**
**74 Strathmore Lane**
**Rockville Centre New York 11570(US)**

(74) Representative: **Henkel, Feiler, Hänzel & Partner**
**Möhlstrasse 37**
**D-8000 München 80(DE)**

(54) **Immunoassay for detection of ras encoded protein.**

(57) This invention provides a method for diagnosing in a subject a neoplastic condition associated with the presence of an activated oncogene which comprises detecting in a sample of a biological fluid from the subject the presence of a polypeptide encoded by the activated oncogene.

A method for diagnosing the presence of a condition in a subject is disclosed which comprises quantitatively determining the amount of an oncogene-encoded polypeptide in a sample from the subject and comparing the amount so determined with the amount determined in a sample from a normal subject.

Further, a method for monitoring the course of a neoplastic condition in a subject is disclosed which comprises quantitatively determining in a first sample of a biological fluid from the subject the presence of an oncogene-encoded polypeptide and comparing the amount so determined with the amount present in a second sample from the subject, such samples being taken at different points in time.

Finally, methods for typing tumors and a method for screening putative therapeutic agents are disclosed.

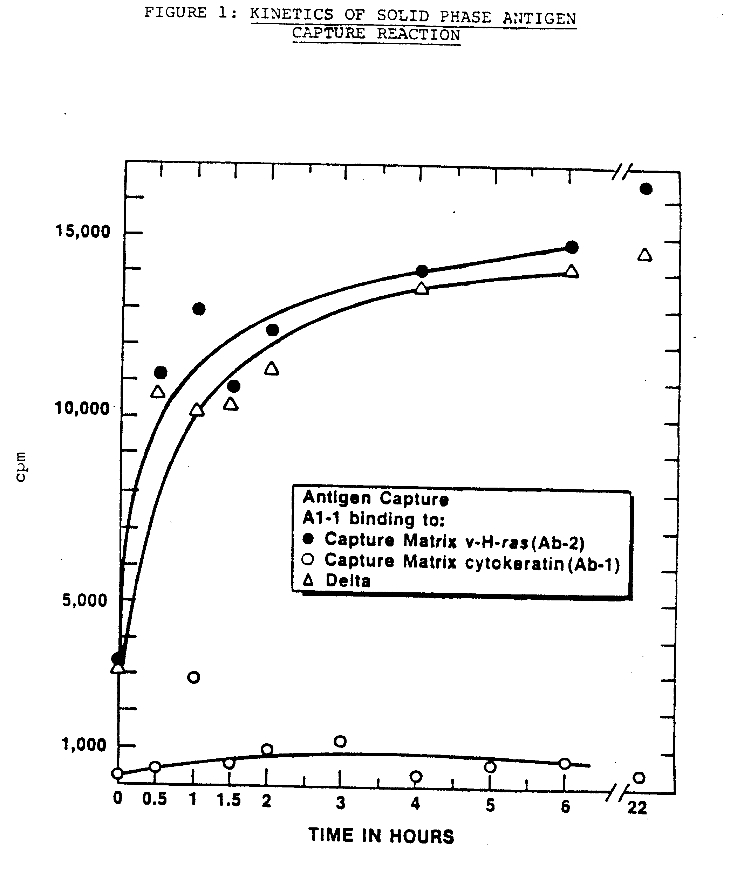

FIGURE 1: KINETICS OF SOLID PHASE ANTIBODY CAPTURE REACTION

Dkt. 23384-A/JPW

IMMUNOASSAY FOR DETECTION OF RAS ENCODED PROTEIN

Throughout this application various publications are referenced by arabic number within parentheses. Full citations for these publications may be found at the end of the specification immediately preceding the claims. The disclosures of these publications in their entireties are hereby incorporated by reference into this application in order to more fully describe the state of the art to which this invention pertains.

Background of the Invention

Certain RNA tumor viruses and human tumor cells contain genomic sequences, termed oncogenes, that encode specific proteins which have been shown to be responsible for the induction of various human and animal cancers (6,18). This invention is based upon the discovery that normally intracellular, oncogene-encoded proteins may be detected in biological fluids such as serum and that such detection may be be utilized to diagnose or monitor neoplastic conditions or states.

DNA isolated from viral or chemically transformed cells has the ability to cause transformation by transfection of normal cell lines. Transforming DNAS have been derived from a variety of human tumors including carcinoma of the bladder, lung, colon, pancreas, sarcomas, hemopoietic tumors, and neuroblastomas (6,20). Discrete transforming DNA sequences have been identified in the DNA of both transformed cells and retroviruses; these sequences are termed oncogenes (16). The oncogenic DNA sequences derived from RNA tumor viruses show great homology with DNA sequences from normal uninfected cells (4). These homologous sequences found in viruses and cells are termed viral oncogenes (v-onc) and cellular oncogenes (c-onc), respectively. The c-onc genes are universally present in the DNA of all individuals and appear to be the evolutionary progenitors of v-onc genes. It is believed that the RNA tumor viruses have acquired oncogenes by recombinational events between the retrovirus and the host genome (transduction) (26).

Cellular oncogenes are normal genes which are conserved throughout evolution and are believed to have normal functional roles in the cell (2). In their "non-cancer inducing state" they are termed proto-oncogenes. The proto-oncogenes are not oncogenic or tumorigenic until they are activated in some way. A number of different genetic mechanisms cause the somatic mutation of normal cellular genes that results in the activated oncogenes found in tumor cells. Mechanisms of oncogene activation include overexpression point mutations, translocations, gene rearrangement, and gene amplification, which may be induced by chemical or physical carcinogenic means, or by the integration of a viral genome adjacent to the proto-oncogene sequences in the host DNA (15).

There are approximately 20 known oncogenes (3,32) and several have been shown to be associated with specific forms of cancer. Oncogenes show sequence homology with normal cellular genes that encode proteins such as growth factors, growth factor membrane receptors, GTP-binding proteins, and specific protein kinases.

Studies have been undertaken to correlate various oncogenes with specific cancer types. The expression of 15 different oncogenes was associated with twenty different tumor types by DNA/RNA hybridization with viral probes (25). The oncogenes (c-onc) c-myc, c-fos, c-H-ras, and c-K-ras were expressed in all tumor tissue examined, while other oncogenes appeared to correlate with specific tumor types. A family of oncogenes found to be most important in the study of human oncogenesis is the ras oncogene. The ras gene family consists of three structurally similar members: H-ras, K-ras, and N-ras. The H- and K-ras transforming genes were first isolated from the Harvey and Kirsten strains of murine sarcoma virus (MuSV). Subsequently, H- and K- ras oncogenes were directly isolated from a variety of human tumor cell lines (by hybridization) and the cloned H- and K-ras genes were found to transform mouse NIH 3T3 cells in culture by transfection (8). The H- and N-ras genes consists of four exons and the K-ras has in addition, an alternative fourth exon (24). The first three exons are more highly conserved than the fourth exon and together they encode a protein of 21,000 daltons, designated p21, comprising 189 amino acids. The p21 has been shown to be structurally and immunologically related to guanine nucleotide binding proteins (10,17).

The p21 protein has been shown by both genetic and biological experimentation to be directly responsible for the ras gene transformation of cells in culture.

The H-ras oncogene isolated from a human bladder carcinoma cell line (T24) was the first oncogene that was shown to differ from the normal H-ras gene by a single base substitution (G to T) which substituted valine for glycine at position twelve in the amino acid sequence (27). The resultant mutant protein was extremely potent in inducing transformation (28). Subsequent studies indicated that mutations corresponding to amino acid changes at positions 12 and 61 led to an activated or transforming ras oncogene (29). These amino acid substitutions produce a ras encoded protein which demonstrates decreased GTPase activity (22). The activated or structurally altered ras oncogene protein has been found in human lung and colon carcinomas (9).

Although most of the evidence implicating the ras gene in human cancer was derived from nucleic acid hybridization studies, there have been several recent studies in which ras p21 protein expression has been demonstrated in human tumors. Hand et al found the ras p21 protein expressed in mammary ductal carcinoma and adenocarcinoma of the colon by immunoperoxidase staining using monoclonal antibodies to the ras protein (13). Similarly, Gallick et al reported the expression of ras encoded p21 in fresh primary and metastatic human colorectal tumors by immunoblotting with peroxidase staining (12). This study showed expression of p21 in early stages of colon tumors and more variable expression in late stage tumors suggesting that tumor progression may lead to the activation of other oncogenes and perhaps growth factors to complement the mutated ras p21.

Presently, the techniques used to show the association of activated ras genes and other oncogenes with neoplasia are oncogene isolation and bioassay, Northern blot- hybridization, and histopathology. Although _ras_ p21 protein is found on the inner aspect of the cell membrane, and therefore is an intracellular protein, it was reasoned that since considerable tissue destruction is involved in the malignant process, _ras_ p21 protein may be released into the sera of cancer patients. The studies described herein are concerned with a sensitive serum-based immunoassay that utilizes monoclonal antibodies to detect _ras_ encoded p21 protein in the sera of a variety of cancer patients but at much higher frequency than in normal human sera. This assay has potential for expansion to other body fluids such as urine and to include all oncogenes associated with the presence of a neoplastic condition.

## Summary of the Invention

The invention concerns a method for diagnosing in a subject a neoplastic condition associated with the presence of an activated oncogene which comprises detecting in a sample of a biological fluid from the subject the presence of at least a portion of a polypeptide encoded by the activated oncogene, the polypeptide normally occurring intracellularly. The detecting may comprise contacting the sample under suitable conditions with a first matrix-bound antibody specific for the polypeptide to form a matrix-first antibody-polypeptide complex, contacting the complex so formed with a second antibody labelled with a detectable marker to form a second complex comprising matrix-first antibody-polypeptide-second antibody, and, finally, detecting the second complex so formed, thereby detecting the presence of the oncogene-encoded polypeptide.

Further, the detecting may comprise contacting the sample under suitable conditions with an antibody specific for the portion of the polypeptide to form an antibody-polypeptide complex and detecting the complex so formed, thereby detecting in the sample the presence of the oncogene-encoded polypeptide.

The detecting may also comprise contacting a detectable control polypeptide with a matrix-bound antibody molecule specific for the portion of the polypeptide in the presence of the oncogene-encoded polypeptide, thereby forming a matrix-antibody-control polypeptide complex, quantitatively determining the number of complexes so formed and comparing the number so determined with the number of complexes so formed in the absence of the oncogene-encoded polypeptide, a decrease in the number of complexes formed indicating the presence of the oncogene-encoded polypeptide in the sample.

This invention also concerns a method for diagnosing in a subject a neoplastic condition associated with the presence of an activated oncogene which comprises contacting the sample under suitable conditions with a first antibody specific for an epitope on the portion of the polypeptide to be detected, and with a second antibody specific for a different epitope on the portion of the polypeptide to be detected so as to form a first antibody-polypeptide-second antibody complex, the complex being detectable.

This invention also concerns a method for diagnosing in a subject a neoplastic condition associated with the presence of an activated oncogene which comprises quantitatively determining in a sample of a biological fluid from the subject the amount of an oncogene-encoded polypeptide and comparing the amount of the polypeptide so determined to the amount in a sample from a normal subject, the presence of a significantly different amount indicating the presence of the neoplastic condition.

The invention also concerns a method for monitoring the course of a neoplastic condition in a subject which comprises quantitatively determining in a first sample of a biological fluid from the subject the presence of an oncogene-encoded polypeptide and comparing the amount so determined with the amount present in a second sample from the subject, such samples being taken at different points in time, a difference in the amounts determined being indicative of the course of the neoplastic condition.

The invention also concerns a method for typing tumors which comprises quantitatively determining in a sample of a biological fluid from a subject with a neoplastic condition the amount of one or more oncogene-encoded polypeptides in the sample, the presence of specific amounts or relative amounts thereof being indicative of a specific tumor type.

The invention also concerns a method for typing tumors which comprises detecting in a sample of a biological fluid from a subject with neoplastic condition the presence of one or more oncogene-encoded polypeptides in the sample, the presence or absence of a specific combination thereof being indicative of a specific tumor type.

The invention also concerns a method for detecting an activated oncogene which comprises detecting in a sample of a biological fluid at least a portion of a polypeptide encoded by the activated oncogene, the polypeptide occurring intracellularly.

Finally, the invention concerns a method for screening putative therapeutic agents for the treatment of a neoplastic condition which comprises quantitatively determining in a first sample from a subject with the neoplastic condition the amount of an oncogene-encoded polypeptide associated with the condition, administering to the subject a therapeutic amount of the agent such that the agent is contacted with a neoplastic cell associated with the condition to produce a treated subject, determining after a suitable period the amount of the polypeptide in a sample from the treated subject, and comparing the amount of polypeptide determined in the first sample with the amount determined in the sample from the treated subject, a significant difference indicating the effectiveness of the agent.

## Brief Description of the Figures

Fig. 1.   Kinetics of The Antigen Capture Reaction.

The capture matrix containing covalently bound v-H-ras (Ab-2) antibody was incubated at 37°C with cellular extract from Al-1 cells for the lengths of time indicated. After each time interval, reporter antibody $^{125}$I-labeled v-H-ras (Ab-1) was incubated with the matrix for 15 hours at 4°C. Capture matrix containing covalently bound cytokeratin (Ab-1) was used as the control capture matrix. The control values (CPM) obtained were subtracted from the sample values to obtain the delta curve. Conditions for the antigen capture procedure are described in detail in "Materials and Methods".

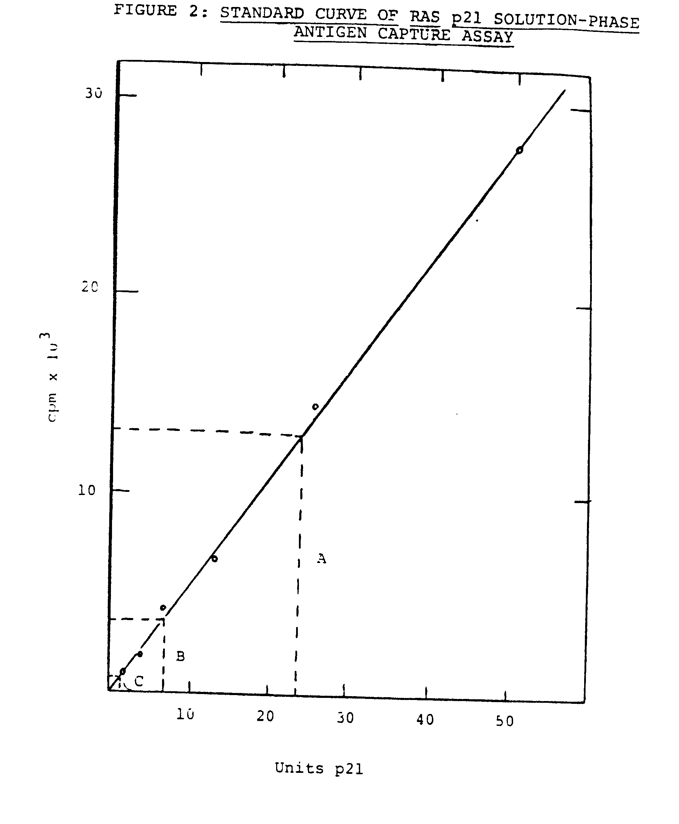

Fig. 2   Standard Curve of ras p21 Solution-Phase Antigen Capture Test.

The test was performed as described in "Materials and Methods" using streptavidin agarose, biotinylated capture antibody, $^{125}$Iodine labeled reporter antibody, and ras p21 antigen diluted into 0.1 ml of normal human sera to generate the standard curve. The internal controls were similarly prepared and then assayed before storage at -70°C; the internal controls contain: A. 23.75, B. 6.25 and C. 1.25 units of ras p21 antigen.

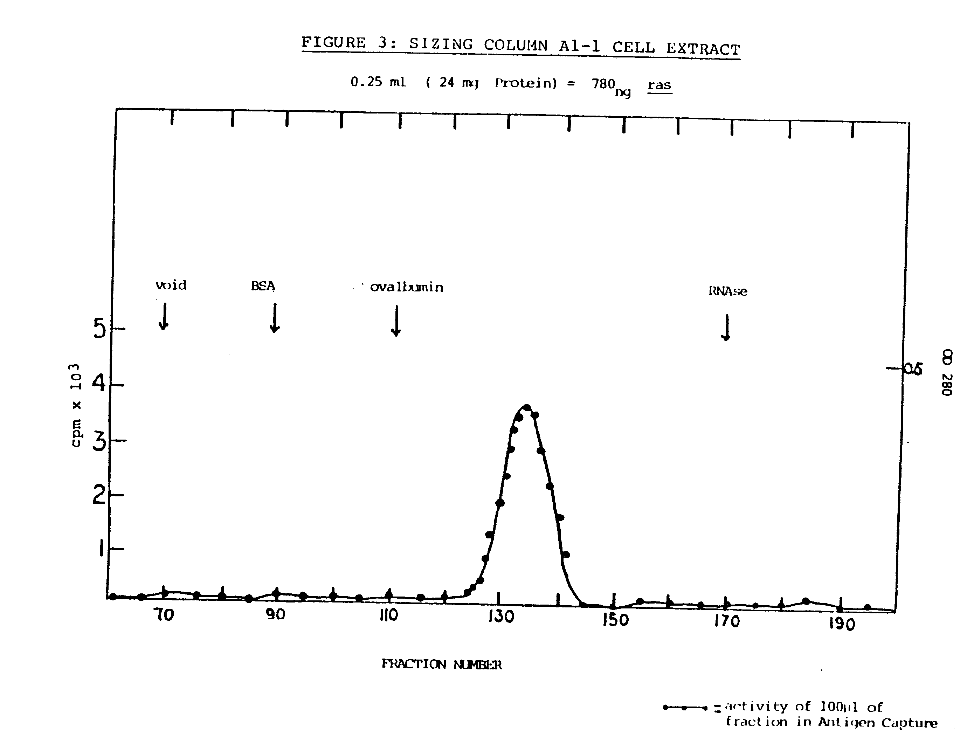

Fig. 3.    Molecular Sizing Analysis of ras p21 Reactivity From Al-1 Cells

Extract of Al-1 cells containing 4 mg total protein in 2 ml was applied to a column of AcA 54 Ultragel (LKB) and the column was developed in buffer containing 10 mM Tris-Acetate (pH 7.5), 1 mM $MgCl_2$, 10 mM NaCl, 0.1 mM sodium EDTA 1.mMDTT and 0.05% octylglucoside (Sigma, St. Louis No. 0-8000). Fractions (4 ml) were collected at $4^{O}C$ and assayed by antigen capture and Western blotting. The internal molecular weight standards were chromatographed separately and locations of elution are shown. The absorbance at 280 nm is shown by the solid line, the antigen capture [125]Iodine CPM values as points.

Fig. 4.    Molecular Sizing Analysis of ras p21 From a Lung Carcinoma Extract of a lung carcinoma containing 4 mg total protein was analyzed as described in the legend to Fig. 3.

Fig. 5     ras p21 Reactivity In Control Sera.

The samples (0.1 ml) of sera from apparently normal humans obtained from Interstate Blood Bank and Clinical Concepts were assayed in duplicate in the solution-phase antigen capture test using streptavidian-agarose to precipitate the complex of biotinylated capture antibody, ras p21 and bound [125]Iodine

reporter antibody as described under "Materials and Methods". The average of the duplicate determinations are shown as solid dots and the mean value of each group is graphed as a solid line.

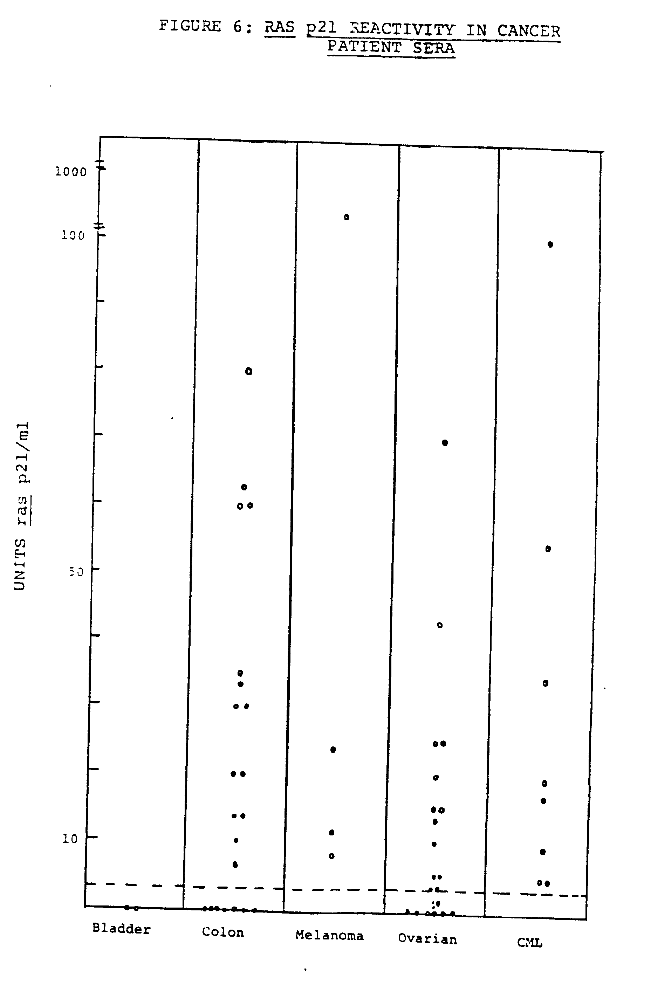

Fig. 6    ras p21 Reactivity in Cancer Patient Sera.

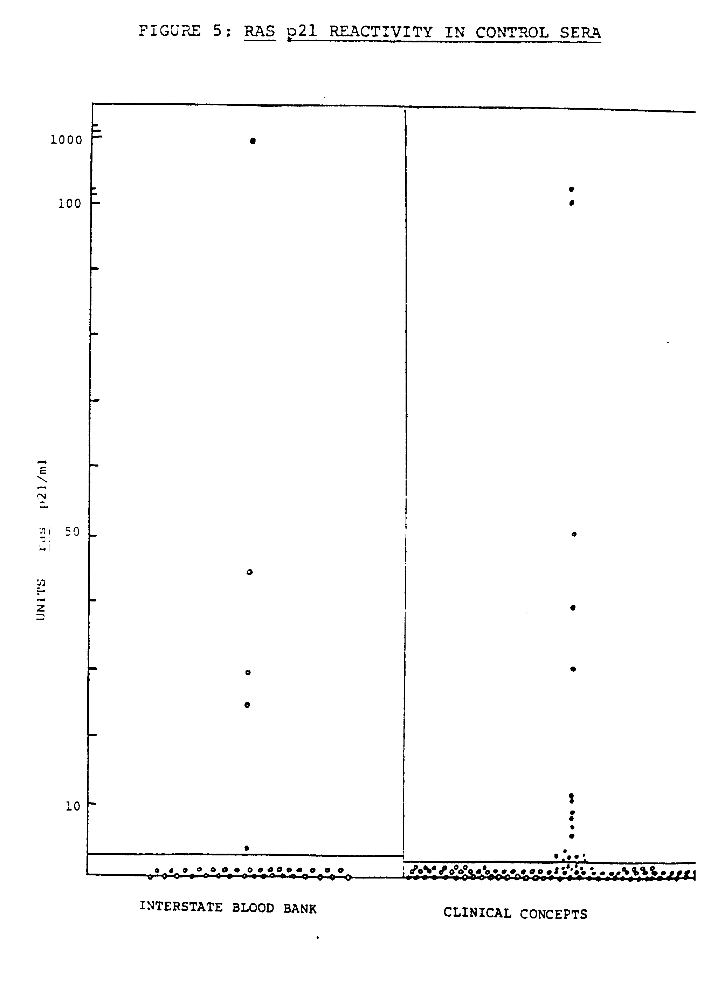

Cancer patient sera (0.1 ml) obtained from the National Cancer Institute or local oncologists were tested in duplicate as described in the legend to Figure 5. The dashed lines represent the mean of the Interstate Blood Bank samples.

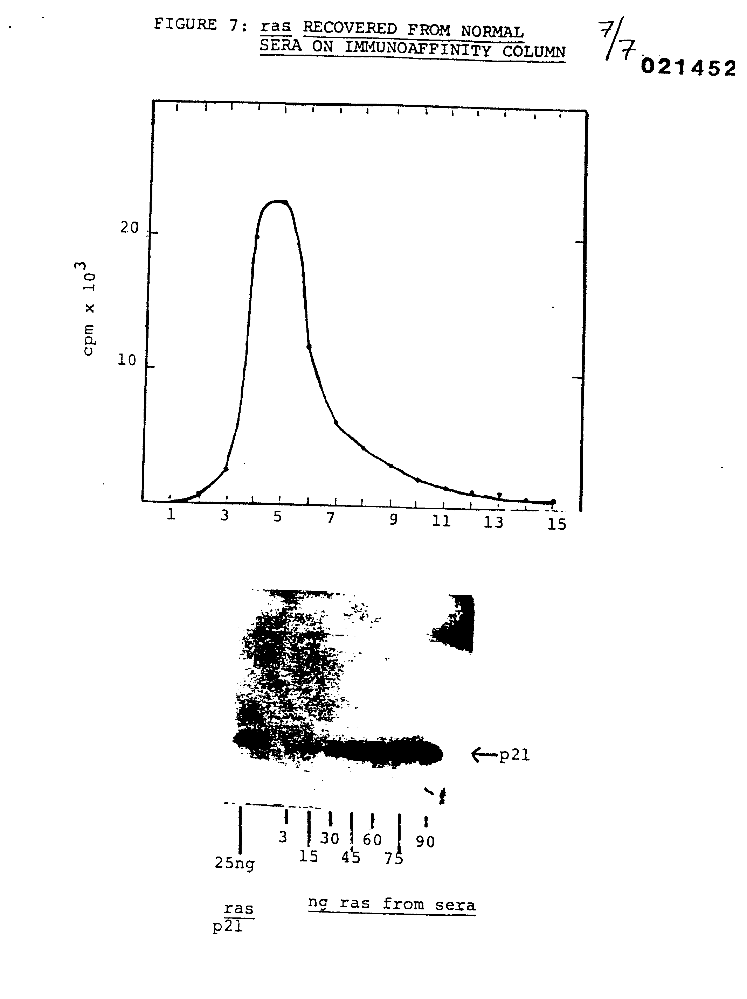

Fig. 7    Recovery of ras p21 from Human Sera The ras reactivity from 40 ml of human sera was isolated by antibody affinity chromatography, as described in "Materials and Methods". The reactivity was located by antigen capture assay pooled and further analyzed by titrating the ras p21 on a Western blot (lower half of Figure 7).

## Detailed Description of the Invention

The invention concerns a method for diagnosing in a subject a neoplastic condition associated with the presence of an activated oncogene. The method comprises detecting in a sample of a biological fluid from the subject, e.g., an animal or a human, the presence of at least a portion of a polypeptide associated with an activated oncogene. The activated oncogene may be c-H-ras, c-K-ras, c-N-ras, c-myc, c-N-myc, c-L-myc, c-R-myc, c-abl, c-fos, or the oncogene which encodes polypeptide p53, although other activated oncogenes may be involved. The polypeptide encoded by the oncogene may be p21 of c-H-ras, c-K-ras or c-N-ras, or may be the polypeptide encoded by c-myc (p62, p65), c-N-myc (p64, p66), phl/c-abl (p210), c-L-myc, c-R-myc, c-fos (p55), or p53, or any other polypeptide which is encoded by an activated oncogene. Further, the polypeptide may be a fusion polypeptide in part encoded by the oncogene and in part by the chromosomal DNA of the subject. The polypeptide may normally be found associated with the inner aspect of the cell membrane or with the nucleus, but may also be an integral membrane protein. In the presently preferred embodiment, the biological fluid is sera; other fluids, e.g., urine, cerebro-spinal fluid, amniotic fluid, sputum, a lung lavage, ascites fluid, saliva, any mucous-type bodily secretion, blood, or plasma may be used.

Various methods may be employed for detecting the presence of the oncogene-encoded polypeptide in the sample. In the presently preferred embodiment, this comprises contacting the sample under suitable conditions with a matrix-bound antibody specific for the polypeptide, thereby forming a matrix-antibody-polypeptide complex.

This complex is further contacted with a second antibody molecule labelled with a detectable marker to form a second complex consisting of matrix-first antibody-polypeptide - second antibody and detecting the second complex so formed thereby detecting the presence of the oncogene-encoded polypeptide. The monoclonal antibodies used were produced by hybridoma cell lines which are fully available from the American Type Culture Collection in Rockville, Maryland, U.S.A. 20852. The matrix-bound antibody may be v-H-ras (Ab-1) produced by hybridoma Y13-259 (ATCC No. CRL1742) and the second antibody may be v-H-ras (Ab-2) produced by hybridoma Y13-238 (ATCC No. CRL1741). In one embodiment, the first antibody is a monoclonal antibody attached to an agarose matrix, e.g., Affi-Gel® 10 (Bio-Rad, Richmond, CA), although the first antibody may be a polyclonal antibody or one of a selected combination (a "cocktail") of antibodies, and may be attached to a Sepharose® (Pharmacia, Piscataway, N.J.) matrix, a tube, a bead or any of a number of support matrices. In the presently preferred embodiment, the second antibody is a monoclonal antibody although it may be a polyclonal antibody, and the attached detectable marker is $^{125}I$, or a variety of other radioactive labels, e.g., cobalt, or any of a variety of colorometric, fluorometric or luminescent markers or may be the product of an enzymatic reaction.

In another embodiment, the detection of the oncogene-encoded polypeptide comprises contacting the sample under suitable conditions with an antibody specific for the portion of the polypeptide, thus forming an antibody-polypeptide complex. The complex so formed is then detected. In a presently preferred embodiment, the antibody is a monoclonal antibody, such antibody forming a detectable immunoprecipitate when contacted with the oncogene-encoded polypeptide. However, the antibody may be monoclonal, polyclonal or one of a selected combination of antibodies. The antibody may also be labelled with a detectable marker, such as $^{125}$I, although the marker may be any one of a variety of radioactive markers, may be a colorometric, fluorometric or luminescent marker, or may be the product of an enzymatic reaction. The antibody may be v-H-ras (Ab-1) produced by hybridoma cell line Y13-259 (ATCC No. CRL1742), which is fully available from the American Type Culture Collection in Rockville, Maryland, U.S.A. 20852.

In a further embodiment, the detection of the polypeptide comprises contacting a detectable control polypeptide with an antibody specific for the portion of the polypeptide in the presence of the oncogene-encoded polypeptide, thus forming a complex comprising antibody-control polypeptide. The number of complexes so formed is quantitatively determined by methods known to those with average skill in the art. The number so determined is compared to the number of complexes formed in the absence of the oncogene-encoded polypeptide, a decrease in the number of complexes formed indicating the presence of the oncogene-encoded polypeptide in the sample. The antibody molecule may be monoclonal, polyclonal or one of a selected combination of antibodies, and may be bound to a matrix such as agarose, Sepharose®, part of a tube or a bead. The control polypeptide may be labelled with a radioactive label, e.g., $^{125}$I, or any of a variety of suitable radioactive labels, or be a colorometric, fluorometric or luminescent marker or be the product of an enzymatic reaction. The antibody may be v-H-ras (Ab-1) produced by hybridoma cell line Y13-259 (ATCC No. CRL1742), which is fully available from the American Type Culture Collection in Rockville, Maryland, U.S.A. 20852.

This invention also concerns a method for diagnosing in a subject a neoplastic condition associated with the presence of an activated oncogene which comprises contacting the sample under suitable conditions with a first antibody specific for an epitope on the portion of the polypeptide to be detected, and with a second antibody specific for a different epitope on the portion of the polypeptide to be detected so as to form a first antibody-polypeptide-second antibody complex, the complex being detectable. Each antibody may be a monoclonal or polyclonal antibody. Detection may be effected by means of an enzymatic reaction, a chemical reaction, a wavelength shift of absorbed light, or an emission of light. The antibodies used were produced by hybridoma cell lines which are fully available from the American Type Culture Collection in Rockville, Maryland, U.S.A. 20852. The first antibody may be v-H-ras (Ab-1) produced by hybridoma Y13-259 (ATCC No. CRL1742) and the second antibody may be v-H-ras (Ab-2) produced by Y13-238 (ATCC No. CRL1741).

A method is disclosed for diagnosing in a subject a neoplastic condition associated with the presence of an activated oncogene. The method comprises quantitatively determining, using techniques known to those skilled in the art, in a sample of a biological fluid from the subject, the amount of an oncogene-encoded polypeptide. The amount so determined is compared to the amount in a sample from a normal subject, e.g., a subject without a tumor, the presence of a significantly different amount indicating the presence of the neoplastic condition.

Further disclosed is a method for monitoring the course of a neoplastic condition in a subject. The method comprises quantitatively determining, by methods known to those skilled in the art, in a first sample of a biological fluid from the subject, the presence of an oncogene-encoded polypeptide. The amount so determined is compared to the amount present in a second sample from the subject, such samples being taken at different points in time, i.e., one sample taken at a sufficient period after the other sample to allow for tumor growth or regression. A difference in the amounts determined is indicative of the course of the neoplastic condition, e.g., growth or regression.

Neoplastic conditions include, but are not limited to carcinomas of the lung, bladder, breast, uterus, prostate, colon, adenocarcinoma of the lung, neuroblastomas, melanomas, rhabdomyosarcomas, lymphomas and leukemias.

Further, a method for typing tumors is disclosed. This method comprises detecting in a sample of a biological fluid from a subject with a neoplastic condition the presence of one or more oncogene-encoded polypeptides, e.g., c-N-ras p21 or p53. The presence or absence of a specific combination may be indicative of a specific tumor type.

A method for typing tumors is also disclosed which comprises quantitatively determining in a sample of a biological fluid from a subject with a neoplastic condition the amount of one or more oncogene-encoded polypeptides, e.g., the amount of c-N-ras p21 or of p53. The presence of specific amounts or relative amounts of the polypeptides, e.g., significant increase in the amount of p53, being indicative of a specific tumor type.

A method for detecting an activated oncogene is also disclosed which comprises detecting in a sample of a biological fluid at least a portion of a polypeptide encoded by the activated oncogene, the polypeptide occurring intracellularly.

Finally, a method for screening putative therapeutic agents for the treatment of a neoplatic condition is disclosed. The method comprises quantitatively determining in a first sample from a subject with the neoplastic condition the amount of an oncogene-encoded polypeptide e.g., c-K-ras p21, associated with the condition. A therapeutic amount of the agent is then administered to the subject such that the agent is contacted with a neoplastic cell associated with the condition to produce a treated subject. After a suitable period, e.g., a period long enough in time to allow significant tumor growth or regression, the amount of the polypeptide in a sample from the treated subject is determined. Finally, the amount of polypeptide determined in the first sample is compared with the amount determined in the sample from the treated subject, a significant difference in the amount of polypeptide from the two samples indicating the effectiveness of the agent, i.e., whether it is associated with tumor regression.

Experimental Details

Materials and Methods

I.  Cell Lines

Hybridoma lines were maintained in RPMI 1640 supplemented with 10% fetal calf serum (MA Bioproducts, Walkersville, MD).  In order to obtain large amounts of immunoglobulin, the cells were injected into pristane (Aldrich) primed rodents and ascites fluid collected after 5-10 days.  Human tumor cell lines were routinely maintained in high glucose Dulbecco's modification of Eagle's medium (MEM) supplemented with 5% fetal calf serum and the transfected, N-, K- or H-ras NIH (3T3) Cl 2.2 cell lines were supplemented with 5% Colorado calf serum (Colorado Serum Co.).  Large scale expansion of the transfected cell lines for ras protein purification was achieved by growing the cells in suspension culture in Eagles minimal essential medium with modified Earle's salts (Gibco, #320-1385) supplemented with 5% Colorado calf serum.  A list of the hybridoma cell lines and their immunoglobulin specificity is shown in Table 1.  The monoclonal antibodies used were produced by hybridoma cell lines which are fully available from the American Type Culture Collection in Rockville, Maryland, U.S.A. 20852.  The antibodies used were v-H-ras (Ab-1) produced by hybridoma Y13-259 (ATCC No. CRL1742) and v-H-ras (Ab-2) produced by hybridoma Y13-238 (ATCC No. CRL1741).  The human tumor cell lines used to detect oncogene protein expression are indicated in Table 2, and Table 3 provides a list of ras transfected, oncogene transformed, viral transformed, and control cell lines.

TABLE 1: <u>HYBRIDOMA CELL LINES AND THEIR</u>
<u>IMMUNOGLOBULIN SPECIFICITY.</u>

| OSI Product Designation | Hybridoma | Origin | Immunoglobulin Specificity | Reference |
|---|---|---|---|---|
| v-H-<u>ras</u> (Ab-1) | Y13-259 | rat | pantropic | Furth <u>et al</u> (1982) J. Virol <u>43</u>: 294. |
| v-H-<u>ras</u> (Ab-2) | Y13-238 | rat | H and K <u>ras</u> p21 | Furth <u>et al</u> (1982) J. Virol <u>43</u>: 294. |
| v-<u>fes</u> (Ab-1) | F113 | rat | v-<u>fes</u> protein | Veronese <u>et al</u> (1982) J. Virol <u>43</u>: 896. |
| v-<u>fms</u> (Ab-1) | SM3 | rat | v-<u>fms</u> protein | Anderson <u>et al</u> (1982) J. Virol <u>44</u>: 696. |
| v-<u>fms</u> (Ab-2) | SM5 | rat | v-<u>fms</u> protein | Anderson <u>et al</u> J. Virol <u>44</u>: 696. |
| p53 (Ab-1) | PAb421 | mouse | p53 tumor antigen | Harlow <u>et al</u> (1981) J. Virol <u>39</u>: 861. |
| cytokeratin (Ab-1) | CK-4 | mouse | cytokeratin-18 | Debus <u>et al</u> (1982) EMBO Journal <u>1</u>, 1641. |

TABLE 2: <u>HUMAN TUMOR CELL LINES</u>

| <u>Cell Line</u> | <u>Tumor Type</u> |
|---|---|
| A431 | epidermoid carcinoma of vulva |
| A375 | melanoma (subcutaneous metastasis) |
| A549 | adeonocarcinoma of lung |
| A204 | rhabdomyosarcoma |
| A673 | rhabdomyosarcoma |
| T-24 | bladder carcinoma |
| HeLa | carcinoma of uterus |
| K562 | erythroleukemia |

TABLE 3: <u>TRANSFECTED, TRANSFORMED, AND</u>
<u>CONTROL CELL LINES</u>

| <u>Cell Line</u> | <u>Description</u> |
|---|---|
| HD-8 | v-H-<u>ras</u> in dog cells |
| mink CC1 64 | control mink |
| GA FeSV mink | v-<u>fes</u> |
| NIH 3T3 Cl 2.2 | Control mouse line used for transfections |
| A1-1 | Transfected with the T-24 H-ras gene encoding Valine at position 12 |
| HL-60-2 | Transfected with the HL-60, N-<u>ras</u> gene encoding Leucine at position 61 |
| OS-8-20-6-2 | Transfected with the Calu-1, K-<u>ras</u> gene encoding cystine at position 12 |

II. Preparation of Cellular Extracts.

Cellular extracts were prepared from Al-1, HD-8, and K562 cells by disruption in a dounce homogenizer with a loose-fitting pestle by 30 strokes. The adherent cells, HD-8 and NIH 3T3 Cl 2.2, were scraped from 150mm tissue culture dishes (Falcon) with a rubber policeman in the presence of phosphate buffered saline (PBS; 10mM sodium phosphate, pH 7.2, 0.9% sodium chloride) containing 1% Triton® X-100 (PBS-Triton, Rohm & Haas, Philadelphia, PA). The cell extracts were centrifuged at 15,000 RPM (Sorvall® RC-5B, SS-34 rotor Ivan Sorvall, Inc., Norwalk, CT) for 10 minutes to remove cellular debris. The cellular extracts were diluted in PBS, and protein concentrations determined by the method of Lowry et al (21).

All cellular extracts were standardized to a concentration of 7.0 mg/ml by lyophilization and reconstitution if the concentration was too low, or by addition of PBS-Triton® if the concentration was too high. These cellular extracts were used as a source of ras p21 protein in the antigen capture procedure.

III. Metabolic Labeling of Cells with $^{35}$S Methionine.

Adherent cell monolayers were incubated for 1 hour at 37°C in Dulbecco's medium (without methionine) supplemented with 5% dialyzed calf serum (Gibco). The media was removed and the cells replenished with fresh media containing 0.2 mCi/ml of $^{35}$S methionine (met) (1075 Ci/mmol; Amersham Corp.) and incubated for two more hours at 37°C as previously described (30). The cells were washed twice with phosphate buffered saline (PBS;

10 mM sodium phosphate, pH 7.20, 0.9% sodium chloride) at 37°C. The cell monolayers were disrupted in PBS containing 1% Triton® X-100, 0.5% sodium deoxycholate and 0.1% sodium dodecyl sulfate (PBSTDS). Cellular extracts were clarified by centrifugation at 30,000 RPM in a Beckman® centrifuge (L8-80M, Beckman Instruments, Inc., Fullerton, CA) equipped with a 70 Ti rotor.

## IV. Purification of Immunoglobulin

Immunoglobulin was purified from tissue culture medium of hybridoma cell lines, ascites fluid, or serum of hyperimmune animals. Serum or ascites fluid was centrifuged at 15,000 RPM for 30 minutes at 4°C (Sorvall® RC-5B centrifuge equipped with a SS-34 rotor) and culture medium from the hybridoma cell lines was centrifuged at 3,000 RPM for 30 minutes at 4°C (Sorvall® RC-3B centrifuged equipped with a H-6,000A rotor). The volumes were recorded and the solution adjusted to final concentration of 20mM Tris-HCl pH 7.8 and 50% saturated ammonium sulfate (385 grams/liter, Schwarz/Mann - enzyme grade). The solution was stirred for 3 hours at 4°C and centrifuged for 30 minutes at 15,000 RPM for sera or ascites fluid (RC-5B), and 3,000 RPM for one hour for the cell culture medium (RC-3B).

The immunoglobulin-containing precipitates were dissolved in PBS containing 0.02% sodium azide, dialyzed against 3 changes of PBS for 15 hours and applied to a column (1.6 x 20 cm, Pharmacia) containing a resin of CM-Affi-Gel® Blue (Bio-Rad, Richmond, CA) equilibrated with PBS. One ml fractions were collected using a SuperRac ( LKB 2211, LKB, Rockville, MD) equipped with a type C collection rack, at a flow rate of 1 ml/min. Fractions were monitored by a Uvicord® S (LKB 2138) spectrophotometer at a wavelength of 280 nm set at an absorbance range of 2.0 AUFS, and recorded by a single channel chart recorder (LKB 2210). Immunoglobulin was found in the unbound effluent fractions as analyzed by SDS - polyacrylamide gel electrophoreseis (SDS-PAGE). Peak fractions were pooled, and the immunoglobulin precipitated by stirring for 3 hours at 4°C in the presence of a final concentration of 50% saturated ammonium sulfate. The solution was centrifuged at 15,000 RPM for 30 minutes at 4°C (Sorvall® RC-5B, SS-34 rotor). Precipitates were dissolved in a minimum volume of PBS and dialyzed in Spectrapor® 2 dialysis tubing (Spectrum Medical Industries, Inc., Los Angeles, CA) for 15 hours at 4°C against 3 changes of DEAE-buffer A (0.02 M Tris-HCl, pH 8.5 containing 0.02% sodium azide). The partially purified immunoglobulin was filtered through a 0.45 mm Millex® filter (Millipore Corp., Bedford, MA) prior to HPLC chromatography. The sample was injected through a 14 ml sample loop (UM 6K sample injector, Waters) onto a TSK DEAE column (150 x 0.5 mm Bio Rad cat 155-0104). A Waters automated gradient controller (Model 680) and pump system (model 510) was utilized for column elution monitored by a variable wavelength detector (Waters Lambda-Max, Model 481) set at 280 nm and a conductivity monitor (Bio-Rad Model #1670440). The sample was eluted in a gradient mode using 0.02 M Tris-HCl pH 8.5 as buffer A and 0.02 M Tris-HCl containing 0.3 M NaCl pH 7.0 as buffer B. Peak Fractions were analyzed for purity by SDS-PAGE.

V. Covalent Coupling of Monoclonal Antibodies to Affi-Gel® 10.

Purified monoclonal antibodies were dialyzed against PBS at 4°C and the Affi-Gel® 10 (Bio-Rad), which had previously been washed with cold distilled $H_2O$, was added to the purified monoclonal antibody solution at a concentration of 7.0mg of protein per ml of Affi-Gel® 10. Generally 70 mg of antibody ligand was added to 10 ml of Affi-Gel® 10. The solution was gently agitated on a Labquake® rotator (Lab Industries #400-10, Lab Industries, Berkeley, CA) for four hours at $4^{o}C$ followed by the addition of ethanolamine (Eastman Kodak) at a final concentration of 0.1 M to block unreacted ester groups. After one hour, the gel matrix was washed extensively with PBS by centrifugation at 2,500 RPM for 10 minutes until the gel was free of reactants as judged by obtaining zero absorbance at 280 nm ($OD_{280}$). For some experiments the antibody ligand for coupling consisted of either v-H-_ras_ (Ab-1) or v-H-_ras_ (Ab-2) and for others both antibodies were coupled to the gel matrix. Monoclonal antibody cytokeratin (Ab-1), prepared against cytokeratin 18 and a second monoclonal antibody v-_fms_ (Ab-2) prepared against v-_fms_ protein were the control ligands coupled to Affi-Gel® 10 at the same protein/gel ratio. Control uncoupled Affi-Gel® 10 was used in designated experiments. To ensure that antibody function was maintained following the coupling procedure, the capture gel matrix was incubated with cell extract containing _ras_ protein and bound protein eluted with sample buffer for analysis by SDS-PAGE.

VI. _Biotinylation of Purified Immunoglobin For Use in the Antigen Capture Procedure._

Monoclonal antibodies were biotinylated according to a protocol distributed by LKB Laboratories. Two hundred microliters of Act BIOTIN solution prepared by adding 2.0 mg of Act BIOTIN to 0.5 ml of anhydrous dimethylformamide (Pierce), was added to 10 mg of immunoglobulin dissolved in 10 ml of 0.2 M sodium bicarbonate pH 8.8 containing 0.15 M NaCl. The reaction was allowed to proceed for 15 minutes at room temperature followed by termination of the reaction with 0.1 ml of 1.0 M ammonium chloride pH 6.0. The biotinylated antibody was dialyzed against PBS to remove other salts and 1.0 ml aliquots stored at $-20^{\circ}$C. To ensure that biological activity of the antibody was maintained following biotinylation, the antibodies were tested by immunoprecipitation of $^{35}$S met labeled cellular extracts containing oncogene encoded proteins. For example v-H-_ras_ (Ab-1) was used to immunoprecipitate _ras_ p21 protein following biotinylation. The v-H-_ras_ (Ab-1) (0.5 μg) was reacted with decreasing volumes of cellular extract containing the _ras_ p21 protein and immunoprecipitated with 0.05 ml of a strepavidin-agarose suspension at 0.24 mg/ml.

VII. <u>Iodination of Purified Immunoglobulin For Use in the Antigen-Capture Procedure.</u>

Aliquots of 20 μl of IODO-GEN® (Pierce Chemical, Rockford, Illinois), which had previously been dissolved in chloroform at a concentration of 10 mg/ml, lyophilized, and kept frozen, were warmed to room temperature. The following were added, in order, to the tube containing IODO GEN®; 0.025 ml of Buffer II (0.4 Tris-HCl, 0.4 mM EDTA, pH 7.4), monoclonal antibody at a concentration of 1.0 mg/.1 ml, and 1.0 mCi of Na$^{125}$I (Amersham® # IMS.40 Amersham International, Buckinghamshire, England). The iodination reaction was allowed to proceed for one minute with gentle shaking and the mixture was subjected to gel filtration chromatography using a G-25 Sephadex® PD10 column (Pharmacia,

Piscataway, N.J.) equilibrated with 10 mM Tris-HCl, 10 mM NaCl, pH 7.8, to remove unreacted free $^{125}$I. One-half ml fractions were collected and 10% trichloroacetic acid (TCA) precipitable counts determined before the samples were pooled.

VIII. Immunoprecipitation of Oncogene-Encoded Proteins with Immunoglobulin.

The $^{35}$S methionine-labeled cellular extract was mixed with 20 ul fetal calf serum per ml final volume, and centrifuged at 3000 RPM for 15 minutes in a table top refrigerated centrifuge (Beckman® TJ-6, Beckman Instruments, Inc., Fullerton, CA). One ml of cell extract was reacted for 15 hours at 4°C with 1 microgram of monoclonal antibody and 50 ul of a 10% (v/v) suspension of Protein-A agarose (BRL) containing 0.175 mg Protein-A. For precipitation using rat monoclonal antibodies, 5 micrograms of goat anti-rat immunoglobulin was also added to bridge the rat immunoglobulin to the Protein-A agarose. The immunocomplexes were washed three times in PBSTDS and collected by centrifugation at 1000 RPM for 10 minutes. The $^{35}$S Met labeled immunoprecipitates were analyzed by electrophoresis using a 5-20% acrylamide SDS-PAGE and subjected to autoradiography.

IX. SDS Polyacrylamide Slab Gel Electrophoresis

Samples were diluted in 20 microliters of sample buffer containing 6.0 M urea (Ultrapure, BRL), 0.1 M Tris-HCl (Sigma; T-1503), pH 6.8, 15% glycerol (Kodak; 114-9939) 2% sodium dodecyl sulfate (Bio-Rad; #116-0302) and 5% B-mercaptoethanol (Bio-Rad; #161-07-10), and electrophoresed on a 5 to 20% acrylamide gradient essentially as described by Laemmli (19). The samples were boiled for 2 minutes prior to application to a 1.5 mm wide slab gel in a Bio-Rad Model 155 Vertical Electrophoresis Cell (Bio-Rad 165-1420) under constant voltage at 45 volts per gel for 15 hours (Hoeffer power supply; PS 1200 DC). Molecular weight standards used were myosin, 200,000; beta-galactosidase, 130,000; phosphorylase B, 92,000; bovine serum albumin, 68,000; ovalbumin, 45,000; carbonic anhydrase, 29,000; soybean trypsin inhibitor, 21,000; lysozyme, 14,400; and cytochrome C, 12,000. Gels were stained with 0.1% Coomassie Blue R-250 (Bio-Rad #16-0400) in 7.0% acetic acid and 10% methanol for five minutes and destained in the same solution without Coomassie. Certain gels were stained by a silver technique as described by Merril (23) (Bio-Rad silver staining kit; #161-0443). Gels containing radioactively labeled samples were subjected to autoradiography as described by Bonner and Laskey (5) using Enhance (New England Nuclear) and type XR-2 X-ray film (Kodak). Molecular weight standards used with gels containing labeled samples were prelabled with [14]C (New England Nuclear).

X.  Solid Phase Antigen Capture Procedure Using Biotinylated Monoclonal Antibodies As Reporter

For each assay point, an aliquot of affinity capture matrix v-H-ras (Ab-1/Ab-2 Affi-Gel® 10) suspension was added to a tube containing 4 mls of PBS with 4% BSA, blocked for one half to three hours, pelleted by centrifugation at 2,800 RPM, then washed once in PBS. The blocked, washed capture matrix was used to probe for p21 in extracts from HD8 cell membranes and serum from cancer patients. Either HD8 cell membrane extract or patient serum was added to each tube containing a pellet of affinity matrix and allowed to react for one to two hours. The matrix was pelleted and washed once in 4.0 mls of PBS containing .05% Tween® 20. One ml of PBS and biotinylated v-H-ras (Ab-1) were added to each tube, incubated at 37°C for 1 hour, pelleted, then washed once in PBS-0.5% Tween® 20 (ICI Americas Inc., Wilmington, DE). An avidin-biotin-horseradish peroxidase complex was prepared by mixing together two drops of solution A (Vectastain®, Avidin Biotin complex (ABC) kit, #PK4004, Vector Laboratories, Burlingame, CA) and 2 drops of solution B in 10.0 ml of PBS containing 0.05% Tween® 20. After 30 minutes, one ml of ABC solution was added to the matrix pellet, mixed, and incubated at 37°C for 30 to 60 minutes. Then matrix was pelleted and washed once with 4.0 mls of PBS-.05% Tween® 20. Developing solution was made as follows: Solution I was prepared by adding 0.06 ml of 30% hydrogen peroxide to 100 ml of PBS. Solution II was prepared by adding 60 mg of horseradish peroxidase developer (Bio Rad) to 20.0 ml of ice-cold methanol. Immediately before use, 5 parts of solution I was mixed with 1 part of solution II and 1.0 ml was added to the matrix. The matrix developed a blue color in samples containing the ras p21 protein.

XI. Solid Phase Antigen Capture Procedure Using Iodinated Monoclonal Antibodies as Reporter Antibodies.

One ml of anti-ras p21 monoclonal v-H-ras (Ab-2) or v-H-ras (Ab-1) capture affinity matrix and the controls cytokeratin (Ab-1) or v-fms (Ab-2) capture matrix were washed two times with 4.0 ml of PBSTDS and one time with 4.0 ml of PBS containing 0.1% BSA (PBS-BSA). Capture matrices used with either cell extracts or human sera were incubated in round bottom (12 x 75 mm, Enkay) or conical polypropylene tubes (12 x 75 mm, Enkay), respectively.

PBS-BSA was added to the gel matrix pellet along with various concentrations of cell extract supernatants as designated in individual experiments (Results). The mixture was either incubated overnight at $4^{O}C$ or for various periods of time at $37^{O}C$ (time course experiment) to optimize the binding of the ras p21 protein to the antigen capture matrix. Following two washes with PBS, 0.1 ml of $^{125}I$ labelled v-H-ras (Ab-1) or v-H-ras (Ab-2) monoclonal antibody (60,000 cpm, 1.0 uCi $^{125}Io$-dine per ug protein) was added, and the mixture incubated for 1.5 hours at $37^{O}C$. The gel matrix was washed one time with PBSTDS and two times with PBS and counted in a gamma counter (LKB #1274 Riagamma).

The gel matrix pellet was suspended in two times its volume of PBS containing 0.1% sodium azide, and 0.01 ml was transferred to a round bottom tube (12 x 75 mm, Enkay) containing a bacterial growth and protease inhibitor solution (BGIPI, 0.05 ml) of final concentrations of 0.005% TPCK (Sigma), 0.01% soybean trypsin inhibitor (Sigma), 0.01% sodium azide, and 0.01% sodium fluoride and 2.75 ul (0.054 TIU) of an aprotinin (Sigma) (19.8 TIU/ml). One half ml of patients or normal human sera (NHS) was added to each tube and the sample was incubated overnight at $4^{O}C$. The gel matrix was washed 2 times with 3.0 ml PBS, and approximately 0.1 ml volume of liquid was retained with the gel matrix pellet. The pellet was rocked for 2 hours at $37^{O}C$ with 0.1 ml of $^{125}I$-labeled monoclonal antibody [v-H-ras (Ab-1) or v-H-ras (Ab-2) - approximately 60,000 CPM, 1.0 uCi $^{125}Iodine$ per 1.0 microgram protein], washed

one time with 3.0 ml of PBS TDS, two times with 3.0 ml PBS, and finally counted in a gamma counter.

XII.    Solution Phase Antigen Capture Test.

An improved solution phase ras p21 antigen capture test which now represents an alternative embodiment of the invention has been formatted. This test requires only 0.1 ml of patient sera, and the other test components consist of strepavidin-agarose (Bethesda Research Laboratories, Rockville, MD, Catalog No. 5942SA, Lab No. 52101), biotinylated capture antibody v-H-ras (Ab-1) and $^{125}$Iodine-labeled v-H-ras (Ab-2) as reporter antibody. The antigen capture configuration is shown diagramatically below:

The conditions for optimized performances have been determined. Sera sample (0.1 ml), 3 micrograms biotinylated capture antibody and 100,000 cpm of $^{125}$Iodine labeled reporter antibody were combined, incubated one hour at $25^{o}$C, and 16 micrograms strepavidin covalently coupled to agarose were added. Following 30 min. of incubation at $25^{o}$ with shaking, the immunocomplexes were collected by centrifugation at 2,800 rpm for 3 min. in a Beckman® refrigerated centrifuge (Model TJ-6), supernatant containing unbound reporter antibody was aspirated and 3 ml of PBS 0.1% Triton® X-100 was added. The centrifugation, aspiration and wash steps were repeated three times, and the bound $^{125}$Iodine counts were measured in a LKB gamma counter (Model 1274).

XIII. <u>Antibody Affinity Chromatography of ras p21.</u>

Monoclonal antibodies with specificity for <u>ras</u> p21 were covalently bound to Tresyl-activated Sepharose® 4B (Pharmacia, Lot. MC01773) as described by the manufacturer. Biological specimens in <u>ras</u> buffer (Tris-HCl 10 mM, pH 7.5, 1mM magnesium chloride, 1 mM dithiothieotol, 0.1 mM, GTP, 0.1% octylglucoside and 0.02% sodium azide) were applied at 4°C, the column was washed with 10 mM Tris-HCl, pH 8.0 buffer containing, 0.5 M sodium chloride until the absorbance at 280 nm reached base line. The <u>ras</u> protein was eluted with 0.1 M sodium citrate at pH 3.5 and individual fractions (3 ml) were immediately neutralized with solid Tris base and 0.3 ml of 10x <u>ras</u> buffer.

XIV. <u>Immunoblotting Analysis.</u>

Samples to be analyzed were first subjected to SDS-PAGE as described on 5-20% acrylamide gradient gels. After electrophoresis, transfer of proteins to nitrocellulose (Schleicher & Schuell, BA 85, 0.45 um) was performed. Proteins were transferred at 70 volts for 2-3 hours at 4°C. Greater than 90% of the <u>ras</u> protein was transferred from the gel as determined by staining the gel with Coomassie Blue after transfer. The nitrocellulose was incubated in 0.5% non-fat powdered milk (Carnation®, Societe des Produits Nestle, S.A., Vevey, Switzerland) in Phosphate buffered saline pH 7.4 (PBS) containing 0.02% sodium azide for 1 hour at room temperature. The filter was then washed with PBS containing 0.1% Tween® 20 (Bio-Rad Lab) and incubated with $^{125}$[I] labeled monoclonal antibody (2.0 x $10^6$ CPM/ml) diluted in the same buffer for 1.5 hours at 37$^{\circ}$C. The filters were washed three times with PBS-Tween® 20, dried and exposed to Kodak XR-2 film at -70$^{\circ}$C using intensifying screens.

Results

I.    Purification of Monoclonal Antibodies.

Monoclonal antibodies to various oncogene encoded proteins were purified from ascites fluid of six different cloned hybridoma cell lines as described in Materials and Methods. Following final purification by HPLC (TSK-DEAE), the immunoglobulin fractions were pooled and their purity assessed by SDS-PAGE. Immunoglobulin was purified from ascites fluid as described in "Materials and Methods" and purity assessed by SDS-PAGE using a 5-20% linear acrylamide gradient. Samples of 30 micrograms, from each pooled immunoglobulin fraction, were applied to the individual lanes. Immunoglobulins were reduced with a final concentration of 5% B-Mercaptoethanol in SDS-PAGE sample buffer. All monoclonal antibodies v-H-ras (Ab-1) pantropic anti-ras p21, v-H-ras (Ab-2) H and K specific anti-ras p21, v-fes (Ab-1), v-fms (Ab-2), p53 (Ab-1), and cytokeratin (Ab-1) were purified to homogeneity and upon reduction with B-mercaptoethanol separated into their respective heavy and light immunoglobulin chains. All the immunoglobulins were of the IgG class except the v-fes (Ab-1) immunoglobulin which was of the IgM class since its heavy chain electrophoresed at 70,000 daltons.

## II. Immunoprecipitation of Oncogene Encoded Proteins.

A series of human tumor cell lines described in Table 2 and mouse cell lines transfected with human ras genes (Table 3) were immunoprecipitated with monoclonal antibodies to detect oncogene encoded proteins. The cell lines were labeled with $^{35}$S-methionine, and prepared as extracts by the procedure described in "Materials and Methods". Immunoglobulin monoclonal antibodies (Mabs), anti-ras p21 (v-H-ras) (Ab-1), p53 (Ab-1), and anti-v-fes (Ab-1) (see Table 1 for immunoglobulin specificity of hybridomas) were purified and used for immunoprecipitation as described in "Materials and Methods". The immunoprecipitates were analyzed by SDS-PAGE and the gels subjected to autoradiography. $^{35}$S methionine labeled cellular extracts from tumor (A431, A375, A549, A204, A673, T-24, HeLa), transfected (A1-1) transformed (GA FeSV mink), and control (mink CC164 NIH 3T3 Cl 2.2) cell lines (described in Tables 2 and 3) were prepared as described in "Materials and Methods". Oncogene encoded proteins were immunoprecipitated, as described in "Materials and Methods". Immunoprecipitates were analyzed by SDS-PAGE using a 5-20% linear acrylamide gradient. Samples were electrophoresed in the presence of SDS-PAGE sample buffer containing 5% B-mercaptoethanol. Gels were stained, destained, treated with Enhance, and exposed to x-ray film. Ras encoded protein was detected in all e cell lines tested as a band migrating at 21,000 daltons, and increased amounts of p21 were evident in the A431 vulva carcinoma and two rhabdomyosarcomas, A204, and A673. Expression of ras protein was greatly enhanced in the T-24 bladder carcinoma derived H-ras gene transfected A1-1 cell line compared to its normal parental counterpart, NIH3T3 Cl 2.2. The p53 protein was detected in A431, A375, A549,

A204, GA FeSV Mink and control mink cells (CCL64), but its expression was not elevated in the other tumor cell lines tested. The transformed cell line GA FeSV mink expressed the p110 fes oncogene protein, while its control untransformed parental cell line, CCL64, did not. These results show readily detectable levels of oncogene protein expression in a variety of human tumor cell lines and which provide a natural source of antigen for the development and evaluation of diagnostic tests for oncogene encoded proteins.

The A1-1, HL60-2, and OS-8-20-6-2 (OS-8) cell lines are derived from the NIH3T3 Cl 2.2 (3T3) mouse fibroblast cell lines transfected with the H-, N-, and K-ras genes, respectively. Further characterization of the antibody specificity of the v-H-ras (Ab-2) and v-H-ras (Ab-1) monoclonal antibodies was determined using these particular cell lines expressing the different ras gene encoded proteins. The specificity of the monoclonal antibodies v-H-ras (Ab-1) and v-H-ras (Ab-2) was determined by their ability to react with the human H, N, or K ras gene encoded protein. The NIH 3T3 Cl 2.2 cells transfected with H-ras, (A1-1), N-ras (HL60-2) and K-ras (OS-8-20-6-2) were $^{35}$S-methionine labeled and ras p21 protein immunoprecipitated by the specific monoclonal antibodies, protein-A agarose (PAA) and goat anti-rat immunoglobulin as detailed in "Materials and Methods":. The v-fes (Ab-1) monoclonal antibody was included as a control. The immunoprecipitates were analyzed by SDS-PAGE, using a 5-20% linear acrylamide gradient, and autoradiography. Samples were electrophoresed in the presence of sample buffer containing 5% B-mercaptoethanol. The v-H-ras (Ab-1) monoclonal antibody immunoprecipitated the H-ras (A1-1), K-ras (OS-8), and N-ras (HL-60-2) ras p21 protein; however, and the v-H-ras (Ab-2) specifically recognized both the H-ras (A1-1) and K-ras (OS-8) p21 protein but not the N-ras (HL-60-2). Thus, the monoclonal antibody v-H-ras (Ab-1) is pantropic in that it recognizes a common determinant on the different ras encoded proteins. The v-H-ras (Ab-2) monoclonal antibody does not precipitate the N-ras gene product and is specific for K- and H-ras. The control v-fes (Ab-1) monoclonal antibody did not immunoprecipitate any p21 from extracts of ras gene transfected cell lines.

III. <u>Retention of Antibody Biological Activity</u> <u>Following Biotinylation or Coupling to</u> <u>Affi-Gel* 10.</u>

Following biotinylation of monoclonal antibodies or coupling of monoclonal antibodies to Affi-Gel* 10, it was necessary to determine if the antibodies were damaged by these procedures. For example, the monoclonal antibody v-H-ras (Ab-1) was biotinylated and tested for its ability to immunoprecipitate ras p21 protein. Analysis of the modified antibody preparations was performed. The function of monoclonal antibody v-H-ras (Ab-1) following biotinylation was assessed by its ability to immunoprecipitate ras p21 in $^{35}$S-labeled methionine cellular extracts (A1-1, T24, HD-8, canine thymus). Increasing amounts of cellular extracts were immunoprecipitated using 0.5 micrograms biotinylated antibody and 50 microliters of strepavidin-agarose suspension at 0.24 mg/ml. The immunoprecipitates were analyzed by SDS-PAGE, using a 5-20% linear acrylamide gradient, and autoradiography. Additionally, the functional capacity of monoclonals v-H-ras (Ab-2) and v-H-ras (Ab-1) coupled to the Affi-Gel* 10 matrix was assessed by its ability to bind ras p21 protein from $^{35}$S-

methionine labeled HD-8 cellular extract. One ml of HD-8 extract was added to increasing amounts of v-H-ras (Ab-1)/v-H-ras (Ab-2) capture matrix and bound ras p21 protein eluted with 100 microliters sample buffer and analyzed by SDS-PAGE, using a 5-20 percent liner gradient and autoradiography. As a control ras p21 protein was immunoprecipitated from 1.0 ml of HD-8 cell extract with 2.0 micrograms of v-H-ras (Ab-1) and 50 microliters of Protein A agarose (PAA). A constant amount of biotinylated antibody (0.5 micrograms) precipitated ras p21 from Al-1, T24, HD-8, and canine thymus cells; the amount of ras p21 detected was proportional to the volume of $^{35}$S methionine labeled cellular extract added to each reaction tube. The ability to immunoprecipitate ras p21 from a variety of cell types in a concentration dependent fashion rules out the possibility that the monoclonal antibodies were damaged by the biotinylation procedure. Similarly, the biological activity of a mixture of v-H-ras (Ab-1) and v-H-ras (Ab-2) was not destroyed by the coupling procedure that covalently linked the antibodies to the Affi-Gel® 10 matrix was evidenced by the ability of the antibody capture matrix to bind ras p21 protein from HD-8 cell extracts. A control of monoclonal antibody v-H-ras (Ab-1) (2.0 micrograms) was also added to the HD-8 extract and the ras p21 protein immunoprecipitated with the addition of Protein-A agarose (50 microliters). A volume of capture matrix, containing 0.5 to 10 micrograms of covalently linked antibody, was added to a constant volume of 1.0 ml of cellular extract. It was found that increasing the amounts of capture matrix added over 1.0 micrograms did not increase the amount of ras p21 protein bound.

IV. Antigen Capture Procedure Using Both v-H-ras (Ab-2) and v-H-ras (Ab-1) Mabs Linked to Affi-Gel® as the Capture Matrix and Biotinylated v-H-ras (Ab-1) as the Reporter Antibody.

A preliminary antigen capture procedure employed biotinylated v-H-ras (Ab-1) as the detector labeling system. In this experiment HD-8 cell membranes were used as a source of ras p21 protein. The cells (1 x $10^7$) were disrupted in hypotonic PBS (diluted 1:4 with water) with a dounce homogenizer and centrifuged at 15,000 RPM for ten minutes at $4^O$C, all as described in Materials and Methods. The cell membranes were suspended in 1.0 ml PBSTDS and incubated with the capture matrix (v-H-ras (Ab-1) and v-H-ras (Ab-2), 0.01 ml) followed by biotinylated v-H-ras (Ab-1) as described in Materials and Methods.

The antigen capture matrix developed a blue color, indicating the presence of ras p21 protein, in the tube containing membrane extract of HD-8 cells but not any controls. Treatment of the gel matrix with 4.0% bovine serum albumin (BSA) before addition of the cell extract caused less of a blue color to develop. Therefore, this treatment decreases some non-specific binding of biotinylated second reporter antibody thus reducing the background. A control of membrane extract incubated with unlinked gel matrix was negative. Also, the second reporter antibody did not bind non-specifically to the v-H-ras (Ab-2)/v-H-ras (Ab-1) antibody linked antigen capture matrix incubated with PBS or PBS-BSA alone. The sera from ten cancer patients with prostate, lung, colon, breast, bladder, or cervical cancer were incubated with antigen capture matrix linked with both v-H-ras (Ab-2) and v-H-ras (Ab-1) monoclonal antibodies as described in "Materials and Methods". Only one patient (pt 5219, breast cancer) was positive as indicated by the blue colored matrix. A sample of normal human sera was negative.

v.    Antigen Capture Procedure using v-H-ras (Ab-2) Mab) Linked to Affi-Gel*, $I^{125}$v-H-ras (Ab-1) as the Reporter Antibody, with Various ras p21-Containing Cellular Extracts.

In order to achieve improved sensitivity of ras p21 detection, the reporter antibody was iodinated. Supernatants, from cellular extracts of HL-60, OS-8, Al-1, 3T3, and HD-8 cell lines were prepared as described in "Materials and Methods". Increasing quantities of cell extract from each cell line were incubated with the antigen capture matrix (v-H-ras (Ab-2) linked Affi-Gel* 10) to optimize the binding of the cell extract to the matrix. The reporter antibody, v-H-ras (Ab-1), was incubated with the cell extract-matrix as described in "Materials and Methods". As seen in Table 4, the anti-ras p21 capture matrix bound 10-20 fold more sample (as reflected by bound reporter v-H-ras (Ab-1) antibody-CPM) than the control matrix of anti-cytokeratin (anti-CK-4) antibody. The control 3T3 cells did not bind to the v-H-ras (Ab-2) complexed affinity matrix and demonstrated approximately the same amount of binding as the ras p21 containing extracts did to the control matrix, thus establishing the relative amount of non-specific binding, or background. Although addition of increasing concentrations of cell extract to the v-H-ras (Ab-2)-matrix showed increased binding, a linear response was not obtained. Almost maximal binding was observed

TABLE 4:  CONCENTRATION DEPENDENCE OF
ras p21 REACTIVITY IN THE
ANTIGEN CAPTURE PROCEDURE
125I COUNTS PER MINUTE (CPM)[a]

| Cell Line[b] | Volume (ml) | Total Protein Assayed (mg) | Capture Matrix v-H-ras (Ab-2)[c] | Capture Matrix Cytokeratin (Ab-1)[d] |
|---|---|---|---|---|
| HL60-2 N-ras | 1.0 | 5.0 | 7115 | 602 |
| | 0.5 | 2.5 | 5097 | 546 |
| | 0.2 | 1.0 | 1324 | 447 |
| | 0.1 | 0.5 | 607 | |
| | 0.5 | 0.2 | 355 | |
| OS-8 K-ras | 1.0 | 5.0 | 8555 | 492 |
| | 0.5 | 2.5 | 11208 | 464 |
| | 0.2 | 1.0 | 7952 | 341 |
| | 0.1 | 0.5 | 6599 | |
| | 0.5 | 0.25 | 3475 | |
| Al-1 H-ras | 1.0 | 2.0 | 13032 | 538 |
| | 0.5 | 1.0 | 5052 | 265 |
| | 0.2 | 0.4 | 8521 | 581 |
| | 0.1 | 0.2 | 4209 | |
| | 0.5 | 0.1 | 3196 | |
| HD-8 H-ras | 1.0 | 1.0 | 7154 | 194 |
| | 0.5 | 0.5 | 6752 | 182 |
| | 0.2 | 0.2 | 3843 | 385 |
| | 0.1 | 0.1 | 1951 | |
| | 0.5 | 0.5 | 1433 | |
| NIH 3T3 Cl 2.2 | 1.0 | 2.0 | 761 | 181 |
| | 0.5 | 1.0 | 318 | 98 |
| | 0.2 | 0.4 | 172 | 89 |
| | 0.1 | 0.2 | 191 | |
| | 0.5 | 0.2 | 214 | |

Table 4: Continued

a) The antigen capture procedure was performed as described for cellular extract supernatants in "Materials and Methods".

b) Cell lines experimentally transformed by H-, K-, or N-ras genes are characterized in Table 3.

c) The capture matrix consisted of v-H-ras (Ab-2) monoclonal antibody covalently linked to Affi-Gel® 10 as described in "Materials and Methods". The reporter antibody used was iodinated v-H-ras (Ab-1) monoclonal antibody.

d) The capture matrix consisted of (cytokeratin (Ab-1) monoclonal antibody covalently linked to Affi-Gel® 10. The reporter antibody used was iodinated v-H-ras (Ab-1) monoclonal antibody.

using approximately 0.5 ml of extract, although in all cases except one (OS-8), 1.0 ml of extract showed slightly higher binding. The addition of increasing concentrations of extract to the control matrix did not show increased binding indicating again that binding to the control matrix was non-specific and also further substantiating that the results obtained with v-H-ras (Ab-2)-matrix indicated specific binding to the p21 ras protein.


VI.  Kinetic Study of ras p21 Binding to v-H-ras (Ab-2) Capture Matrix.

Supernatant extract from Al-1 (2.1 mg/ml) cells (100 ul) were incubated with the v-H-ras (Ab-2)-capture matrix and the control cytokeratin (Ab-1) capture matrix, for times ranging from 30 minutes to 22 hours. At the end of each time period, $^{125}I$-v-H-ras (Ab-1) (40,000 CPM/0.1 ml) anti-ras p21 was added and incubated with the cell extract-matrix mixture overnight at $4^{\circ}C$. Figure 1 shows that a maximum amount of binding of cell extracts to the antigen capture matrix was achieved within 30 minutes. Again the v-H-ras (Ab-2) capture matrix showed approximately a 10-20 fold increase in binding over the control cytokeratin (Ab-1) matrix.

VII.  Antigen Capture Procedure Using Serum of Cancer Patients, Non-Cancer Patients, and Normal Donors.

The antigen capture procedure was performed using v-H-ras (Ab-2) capture affinity matrix and $^{125}I$-v-H-ras (Ab-1) reporter antibody as described in "Materials and Methods". Table 5 is a summary of up to four separate

TABLE 5: <u>THE USE OF THE ANTIGEN CAPTURE PROCEDURE TO DETECT ras p21 PROTEIN</u>

<u>IN THE SERA OF CANCER PATIENTS</u>

$I^{125}$ COUNTS PER MINUTE (CPM)

| Tumor Type[b] | Serum Number | Capture Matrix v-H-ras (Ab-2)[c] | | | | | Capture Matrix Cytokeratin (Ab-1)[d] |
|---|---|---|---|---|---|---|---|
| | | Exp1 | Exp2 | Exp3 | Exp4 | Mean | |
| Control NIIS | 849 | ND | 686 | ND | ND | 686 | 211[e] |
| Control NIIS | 883 | 371 | 585 | 658 | ND | 538 | 327[e] |
| Control NIIS | 884 | 374 | 302 | ND | ND | 338 | 215[e] |
| Control NHP | 5140 | 511 | 495 | 273 | ND | 503 | 626[e] |
| CML | 5187 | 9942 | ND | ND | ND | 9942 | ND |
| CML | 5189 | 6216 | 5899 | ND | ND | 6057 | ND |
| CML | 5190 | 1278 | 1348 | ND | ND | 1313 | ND |
| CML | 5200 | 918 | 1873 | 2099 | 1951 | 1710 | 247 |
| CML | 5205 | 839g | 429 | 488 | 470 | 539 | 186 |
| CML | 5295-72 | 268 | 223 | 242 | ND | 244 | 147 |
| Bladder | 5279 | 301 | ND | ND | ND | .301 | ND |
| Bladder | 5283 | 6376 | 7180 | ND | ND | 6778 | ND |
| Bladder | 5295-10 | 552 | ND | ND | ND | 552 | ND |
| Bladder | 5295-13 | 538 | ND | ND | ND | 538 | ND |
| Bladder | 5295-14 | 567 | 666 | ND | 490 | 574 | ND |

## I[125] COUNTS PER MINUTE (CPM)

| Tumor Type[b] | Serum Number | Capture Matrix v-H-_ras_ (Ab-2)[c] | | | | | Capture Matrix Cytokeratin (Ab-1)[d] |
|---|---|---|---|---|---|---|---|
| | | Exp1 | Exp2 | Exp3 | Exp4 | Mean | |
| Bladder | 5295-15 | 1308 | 1612 | 1192 | 924 | 1259 | 487 |
| Bladder | 5295-16 | 2029 | 2992 | 3190 | 1852 | 2515 | 4983 [f] |
| Bladder | 5295-35 | 731 | ND | 653 | ND | 692 | ND |
| Bladder | 5295-36 | 1569 | 2479 | 1630 | 1468 | 1786 | ND |
| Prostate | 5177 | 428 | ND | ND | ND | 428 | ND |
| Prostate | 5179 | 413 | ND | ND | ND | 413 | ND |
| Prostate | 5184 | 339 | ND | ND | ND | 339 | ND |
| Prostate | 5231 | 1380 | 1050 | ND | ND | 1215 | ND |
| Prostate | 5232 | 1045 | 1260 | ND | ND | 1152 | ND |
| Prostate | 5238 | 545 | ND | ND | ND | 545 | ND |
| Prostate | 5242 | 1779 | ND | ND | ND | 1779 | ND |
| Breast | 5112 | 926 | ND | ND | ND | 926 | ND |
| Breast | 5113 | 243 | 395 | ND | ND | 319 | ND |
| Breast | 5165 | 119 | 266 | ND | ND | 192 | ND |
| Breast | 5166 | 142 | 200 | ND | ND | 171 | ND |

TABLE 5: Continued

$I^{125}$ COUNTS PER MINUTE (CPM)

| Tumor Type[b] | Serum Number | Capture Matrix v-H-ras (Ab-2)[c] | | | | | Capture Matrix Cytokeratin (Ab-1)[d] |
|---|---|---|---|---|---|---|---|
| | | Exp1 | Exp2 | Exp3 | Exp4 | Mean | |
| Breast | 5219 | 11373[h] | 7377 | ND | ND | 9375 | ND |
| Breast | 5222 | 1342 | 1319 | ND | ND | 1330 | ND |
| Breast | 5269 | 269 | ND | ND | ND | 269 | ND |
| Breast | 5285 | 190 | ND | ND | ND | 190 | ND |
| Breast | 5295-4 | 150 | 106 | 114 | 108 | 119 | 108 |
| Breast | 5295-18 | 999 | ND | ND | ND | 999 | ND |
| Breast | 5295-19 | 1633 | ND | ND | ND | 1633 | ND |
| Breast | 5295-24 | 250 | 155 | ND | ND | 202 | ND |
| Lung | 5115 | 4575 | 6686 | 5503 | ND | 5588 | ND |
| Lung | 5116 | 1834 | 1900 | 2318 | 1538 | 1897 | ND |
| Lung | 5123 | 312 | ND | ND | ND | 312 | ND |
| Lung | 5127 | 226 | 175 | ND | ND | 200 | ND |
| Lung | 5249 | 467 | ND | ND | ND | 467 | ND |
| Lung | 5250 | 394 | ND | ND | ND | 394 | ND |
| Lung | 5272 | 611 | ND | ND | ND | 611 | ND |
| Lung | 5284 | 171 | ND | ND | ND | 171 | ND |

$I^{125}$ COUNTS PER MINUTE (CPM)

| | | Capture Matrix v-H-ras (Ab-2)[c] | | | | | Capture Matrix Cytokeratin |
|---|---|---|---|---|---|---|---|
| Tumor Type[b] | Serum Number | Exp1 | Exp2 | Exp3 | Exp4 | Mean | (Ab-1)[d] |
| Lung | 5295-60 | 273 | ND | ND | ND | 273 | ND |
| Lung | 5295-61 | 716 | 906 | 618 | 896 | 784 | 143 |
| Lung | 5295-69 | 453 | ND | ND | ND | 453 | ND |
| Lung | 5295-70 | 343 | 389 | 128 | ND | 286 | ND |
| Lung | 5295-71 | 434 | ND | ND | ND | 434 | ND |
| Colon | 5057 | 48 | 58 | ND | ND | 53 | ND |
| Colon | 5061 | 57 | ND | ND | ND | 57 | ND |
| Colon | 5062 | 161 | ND | ND | ND | 161 | ND |
| Colon | 5066 | 91 | ND | ND | ND | 91 | ND |
| Colon | 5118 | 324 | 181 | ND | ND | 252 | ND |
| Colon | 5120 | 297 | 563 | ND | ND | 430 | ND |
| Colon | 5287 | 301 | ND | ND | ND | 301 | ND |
| Colon | 5295-27 | 900 | ND | ND | ND | 900 | ND |
| Colon | 5295-100 | 447 | ND | ND | ND | 447 | ND |
| Colon | 5295-63 | 414 | ND | ND | ND | 414 | ND |
| Brain | 5280 | 389 | ND | ND | ND | 389 | ND |
| Brain | 5289 | 613 | ND | ND | ND | 613 | ND |

TABLE 5: Continued

$I^{125}$ COUNTS PER MINUTE (CPM)

| Tumor Type[b] | Serum Number | Capture Matrix v-H-ras (Ab-2)[c] | | | | | Capture Matrix Cytokeratin (Ab-1)[d] |
|---|---|---|---|---|---|---|---|
| | | Exp1 | Exp2 | Exp3 | Exp4 | Mean | |
| Tongue | 5278 | 5354 | ND | ND | ND | 5354 | ND |
| Ovarian | 5295-39 | 501 | ND | ND | ND | 501 | ND |
| Ovarian | 5229 | 341 | ND | ND | ND | 341 | ND |
| Esophagus | 5271 | 157 | ND | ND | ND | 157 | ND |
| Larynx | 5156 | 411 | ND | ND | ND | 411 | ND |
| Larynx | 5270 | 175 | ND | ND | ND | 175 | ND |
| Oat Cell Ca of Lung | 5209 | 284 | ND | ND | ND | 284 | ND |
| Cervix | 5153 | 148 | ND | ND | ND | 148 | ND |
| Vulva | 5141 | 313 | ND | ND | ND | 313 | ND |
| Renal Cell | 5133 | 329 | ND | ND | ND | 329 | ND |
| Multiple Myeloma | 5125 | 174 | ND | ND | ND | 174 | ND |

I$^{125}$ COUNTS PER MINUTE (CPM)

| Tumor Type[b] | Serum Number | Capture Matrix v-H-ras (Ab-2)[c] | | | | | Capture Matrix Cytokeratin (Ab-1)[d] |
| | | Exp1 | Exp2 | Exp3 | Exp4 | Mean | |
|---|---|---|---|---|---|---|---|
| **NON-NEOPLASTIC CONDITIONS** | | | | | | | |
| GI Polyp | 5040 | 215 | ND | ND | ND | 215 | ND |
| GI Polyp | 5043 | 92 | ND | ND | ND | 92 | ND |
| GI Polyp | 5044 | 280 | ND | ND | ND | 280 | ND |
| GI Polyp | 5045 | 112 | ND | ND | ND | 112 | ND |
| Dysmyelopoetic Syndrome | 5126 | 453 | ND | ND | ND | 453 | ND |
| Cholecystectomy | 5135 | 258 | ND | ND | ND | 258 | ND |
| Pregnancy | 5114 | 363 | ND | ND | ND | 363 | ND |

a) The antigen capture procedure was performed as described for human sera in "Materials and Methods".

b) The sera from patients with tumors was obtained from the National Institutes of Health, Biological Carcinogenesis Branch, Bethesda, Maryland, and control sera and plasma was obtained from normal available individuals.

TABLE 5:  Continued

c)  The capture matrix consisted of v-H-ras (Ab-2) monoclonal antibody linked to Affi-Gel®10 as described "Materials and Methods".  The reporter antibody used was iodinated v-H-ras (Ab-1) monoclonal antibody.

d)  The capture matrix consisted of cytokeratin (Ab-1) monoclonal antibody covalently linked to Affi-Gel®10.  The reporter antibody used was iodinated v-H-ras (Ab-1) monoclonal antibody.

e)  The first four numbers are an average of two determinations.

f)  A cancer patient which was positive using the control cytokeratin (Ab-1) capture matrix.

g)  A cancer patient that was originally minimally positive and became negative in subsequent tests.

h)  A patient with breast cancer demonstrating very high counts (CPM's) was also positive using biotinylated antibody as reporter (see Section IV. - results).

antigen capture experiments on the sera of a total of 70 cancer patients. Sera obtained from patients with non-neoplastic conditions are listed at the end of the table. Counts (CPM) higher than 700 were obtained in 22 out of 70 sera from cancer patients and the sera samples from non-cancer patients and normal donors were all below 700. The ras p21 protein was ostensibly detected in approximately 33% of patients sera examined. The state of the patient's malignancy was not adequately recorded at the time the serum sample was taken; therefore, those negative for ras p21 may be due to sera collected following tumor extirpation or therapy. Alternatively, negative results may be due to lack of cross-reactivity of the monoclonal anti-ras antibody to any of the determinants present on ras protein in any one cancer patient's serum. An important point to be noted from the data presented in Table 5 is that the antigen capture procedure appears to be a reliable test as observed by the consistency in the experimental results obtained upon repeated testing of the sera samples. One patient's sera (#5205) that was originally minimally positive, became negative in three subsequent experiments. Four normal control sera and six out of seven cancer patients' sera were negative in the antigen capture procedure employing the cytokeratin (Ab-1) monoclonal antibody as the control capture matrix. The sera from a patient with breast cancer (#5219) that demonstrated one of the highest average counts CPM (Table 5) was the same and only patient that tested positive using biotinylated v-H-ras (Ab-1) as the detector antibody.     Apparently,   using   a biotinylated - peroxidase reporter antibody system, the ras protein can only be detected in the sera of patients with elevated levels of the protein. Thus, a radiolabeled reporter system provided the increased sensitivity necessary to detect the ras protein in a number of patients sera containing lower levels of the protein. The specific types of cancer that shared the highest detectable levels of ras protein, or reflected by counts obtained with their sera, is reported in descending order: CML, bladder, prostate, breast, lung, and colon.


VIII.   Comparison of a Reverse Configuration of Anti-ras Antibody   Presentation   for   Capture   Matrix   and Reported Antibody in the Antigen Capture Procedure Using Cellular Extracts.


Separate antigen capture matrices linked to the puri- fied anti-ras p21 monoclonal antibodies of either v-H- ras (Ab-2) or v-H-ras (Ab-1), and separately labeled iodinated ($^{125}$I) v-H-ras (Ab-2) and v-H-ras (Ab-1) antibodies were used in an experiment to determine the order of antibody presentation that would provide the greatest assay sensitivity. Supernatant extracts of Al-1, OS-8, HL-60-2, and control NIH 3T3 Cl 2.2 cell extracts were incubated with both the "original" configuration of antibody presentation, v-H-ras (Ab-2) capture matrix and $^{125}$I-v-H-ras (Ab-1) reporter antibody, and the "reverse" configuration v-H-ras (Ab-1) capture matrix and $^{125}$I-v-H-ras (Ab-2) reporter antibody.   The control cytokeratin (Ab-1) capture matrix was used separately with both antibodies $^{125}$I-v-H-ras (Ab-2) and $^{125}$I-v-H-ras (Ab-1) as reporter group. Cell extracts were incubated with the capture matrices for 2.5 hours at 37°C followed by the standard washes described in "Materials and Methods"; the $^{125}$I reporter antibody was added and incubation was carried out overnight at 4°C. A summary of these results is shown in Table 6. There was a 2.1, 2.4 and 4.0 fold increase in detection of ras p21 in the cellular extracts Al-1, OS-8, and HL-60-2 respectively using the reverse configuration of antibody presentation, v-H-ras (Ab-1) capture matrix, v-H-ras (Ab-2) reporter than the previously used v-H-ras (Ab-2) capture matrix and v-H-ras (Ab-1) reporter monoclonal antibody. Thus, reversing the presentation of antibody greatly increased the sensitivity for the detection of N-ras p21. Although a four fold increase in binding (CPM) was also observed using the control capture matrix cytokeratin (Ab-1) and the $^{125}$I-v-H-ras (Ab-2) reporter antibody, after subtraction of the control background from the test samples, a net increase was still observed. The binding of extracts of the 3T3 cells to the anti-ras capture matrix was 10-20 fold less than the ras transfected counterparts. The Al-1 cell extract, used as a control, did not show appreciable binding to the control cytokeratin (Ab-1) affinity matrix.

IX. **Comparison of a Reverse Configuration and Anti-ras Antibody Presentation for Capture Matrix and Reporter Antibody in the Antigen Capture Procedure Using Human Sera.**

The sera samples from 18 cancer patients and two normals, which were previously tested using v-H-ras (Ab-2)-matrix and v-H-ras (Ab-1) reporter antibody were

$^{125}$I COUNTS PER MINUTE (CPM)

| Cellular Extract[b] (Supernatant) | Protein(mg) | Capture Matrix v-H-ras (Ab-1) $^{125}$I Reporter v-H-ras (Ab-1)[c] | Capture Matrix v-H-ras (Ab-1) $^{125}$I Reporter v-H-ras (Ab-2)[c] |
|---|---|---|---|
| Al-1 (H-ras) | 105 | 6927 | 14900 |
| OS8 (K-ras | 500 | 8120 | 19154 |
| HL60-2 (N-ras) | 1000 | 3760 | 14926 |
| NIH 3T3 Cl 2.2 | 200 | 280 | 1185 |

a)    The antigen capture procedure was performed as described for cellular extract supernatants in "Materials and Methods".

b)    Cell lines transfected with H-, K-, or N-ras genes are charcterized in Table 3.

c)    The preparation of capture matrices and iodinated reporter antibodies are described in detail in "Materials and Methods".

retested using the reverse configuration of antibody presentation as described above. The data is presented in Table 7. Approximately a three-fold increase in sensitivity for ras p21 detection in sera was found using the reverse configuration and, with this increase in sensitivity, four samples that were previously negative became positive. One sample that was positive became negative.

Antigen capture reactivity was detected in four sera samples which were previously negative using the "original" configuration of antibody presentation. One can speculate that the use of the pantropic antisera (v-H-ras (Ab-1)) as the capture matrix may provide a more general recognition of a greater range of ras haplotypes present in a variety of sera; however, the limitation of detection is ultimately dependent on the recognition of ras protein by the reporter antibody used. In this way, the antigen capture procedure can be designed to be either specific for a particular ras protein or more general depending on the specificities of the antibodies used and the order of their presentation.

In order to understand and to improve specific details involved in test design and protocol for the antigen capture procedure, a series of experiments was performed using various permutations of antibody presentation. The v-H-ras (Ab-1), v-H-ras (Ab-2), v-fms (Ab-2) and cytokeratin (Ab-1) were used as antibodies linked to capture matrices and v-H-ras (Ab-1), v-H-ras (Ab-2), and v-fms (Ab-2) were used as reporter antibodies. A constant amount of normal human sera (0.5 ml) was compared to this same sera, containing 0.05 ml of cellular extract from A1-1 cells (7.0 mg/ml total protein

TABLE 7: COMPARISON OF THE REVERSAL OF ANTIGEN CAPTURE AND REPORTER ANTIBODY PRESENTATION ON THE DETECTION OF ras p21 IN THE SERA OF CANCER PATIENTS[a]

$^{125}$I COUNTS PER MINUTE (CPM)

| Tumor Type | Serum Number[b] | ORIGINAL CONFIGURATION: Capture Matrix v-H-ras (Ab-2) Reporter $^{125}$I v-H-ras (Ab-1)[c,e] | ORIGINAL CONFIGURATION: Capture Matrix v-H-ras (Ab-2) Reporter $^{125}$I v-H-ras (Ab-1)[c,d] | REVERSED CONFIGURATION: Capture Matrix v-H-ras (Ab-1) Reporter $^{125}$I v-H-ras (Ab-2)[c,d] |
|---|---|---|---|---|
| Control NHS | | ND | ND | 1703 |
| Control NHP | | ND | ND | 708 |
| Lung | 5115 | 5630 | 5503 | 17128 |
| Lung | 5116 | 2017 | 1538 | 2349 |
| CML | 5189 | 6216 | 5899 | 12051 |
| CML | 5205 | 839 | 543 | 4093[f] |
| Breast | 5219 | 11373 | 7377 | 6083 |
| Breast | 5222 | 1342 | 1319 | 1792[g] |

TABLE 7: Continued

| Tumor Type | Serum Number[b] | ORIGINAL CONFIGURATION: Capture Matrix v-H-ras (Ab-2) Reporter $^{125}$I v-H-ras (Ab-1)[c,e] | ORIGINAL CONFIGURATION: Capture Matrix v-H-ras (Ab-2) Reporter $^{125}$I v-H-ras (Ab-1)[c,d] | REVERSED CONFIGURATION: Capture Matrix v-H-ras (Ab-1) Reporter $^{125}$I v-H-ras (Ab-2)[c,d] |
|---|---|---|---|---|
| Prostate | 5232 | 1045 | 1260 | 1947 |
| Bladder | 5295-35 | 731 | 653 | 3825[f] |
| Bladder | 5295-36 | 1892 | 1468 | 10991 |
| Colon | 5118 | 324 | 181 | 1270 |
| Lung | 5127 | 226 | 175 | 518 |
| Lung | 5295-70 | 365 | 128 | 711 |
| Larynx | 5270 | 175 | 88 | 818 |
| Colon | 5057 | 58 | 48 | 99 |
| Colon | 5295-63 | 414 | ND | 4077[f] |
| Breast | 5295-24 | 155 | 250 | 356 |
| Breast | 5166 | 200 | 142 | 158 |
| Bladder | 5295-14 | 490 | 617 | 2374[f] |

Table 7: Continued

a)  The antigen capture procedure was performed as described for human sera in "Materials and Methods".

b)  The sera from patients with tumors was obtained from the National Institutes of Health, Biological Carcinogenesis Branch, Bethesda, Maryland.  Control sera and control plasma were obtained from normal individuals.

c)  The preparation of capture matrices and iodinated reporter antibodies are described in "Materials and Methods".

d)  Data from experiments performed simultaneously.

e)  Average of three previous experiments.

f)  Sera samples that became positive using the reversed configuration.

g)  Serum sample that became negative using reverse configuration; this sample was considered to be negative on the basis that it was below the highest control value (1703).

lot #A106) as a source of ras p21 protein, utilizing the antibody configurations presented in Table 8. The following possible conclusions can be derived from this data:

1. The ras p21 protein was not detected in sera containing the protein if v-H-ras (Ab-1) was used as both capture matrix and reporter antibody (506 CPM), however, if v-H-ras (Ab-2) was used as a capture matrix and v-H-ras (Ab-1) was used as reporter antibody a 10-fold increase in the level of ras p21 detection was observed (5,825 CPM). This same 10-fold increase in counts was seen if v-H-ras (Ab-1) was used as capture matrix and v-H-ras (Ab-2) used as reporter (10,810 CPM) compared to v-H-ras (Ab-2) as both capture matrix and reporter antibody (1,660 CPM). It can be reasoned that using the same monoclonal antibody, which is directed toward only one epitope of the antigen, in a two step procedure, can preclude the recognition and binding of that antibody to the antigen in a second step (reporter antibody), because all the sites on the antigen are bound and blocked for further recognition during the first step. Moreover, another monoclonal antibody directed against a different epitope cf the antigen, or a polyclonal, would be able to recognize and bind in a second step providing the two epitopes were not physically adjacent so that one would sterically hinder the other from subsequently binding.

2. The v-H-ras (Ab-2) monoclonal antibody used for both capture matrix and reporter showed higher counts (1,660) than the v-H-ras (Ab-1) monoclonal antibody (506). The v-H-ras (Ab-2) may demonstrate some non-specific binding thereby increasing the background counts obtained.

$^{125}$I – COUNTS PER MINUTE (CPM)

CAPTURE MATRICES

| $^{125}$I labeled Reporter Antibody[c] | v-H-ras (Ab-1)[b] | | v-H-ras (Ab-2)[b] | | v-fms (Ab-2)[b] | | Cytokeratin (Ab-1)[b] | |
|---|---|---|---|---|---|---|---|---|
| | NHS | NHS& Al-1[d] | NHS | NHS& Al-1[d] | NHS | NHS& Al-1[d] | NHS | NHS& Al-1[d] |
| v-H-ras (Ab-1) | 563 | 506 | 503 | 5,825 | 433 | 359 | 463 | 1,106 |
| v-H-ras (Ab-2) | 591 | 10,810 | 329 | 1,660 | 345 | 2,821 | 316 | 2,073 |
| v-fms (Ab-2) | 196 | 178 | 161 | 183 | 209 | 148 | 272 | 168 |

a) The antigen capture procedure was performed as described for human sera in "Materials and Methods".

b) The capture matrices were prepared using monoclonal antibodies v-H-ras (Ab-1), v-H-ras (Ab-2), v-fms (Ab-2), and cytokeratin (Ab-1) exactly as described in "Materials and Methods".

c) The reporter monoclonal antibodies v-H-ras (Ab-1), v-H-ras (Ab-2), and v-fms (Ab-2) were iodinated as described in "Materials and Methods".

d) Normal human sera was obtained from a commercial blood bank. Each sample received 0.05 ml of a cellular extract of Al-1 cells (7.0 mg/ml total protein (lot #A106) as a source of ras p21 protein.

3. Using the "reverse configuration" of v-H-ras (Ab-1) as capture matrix and v-H-ras (Ab-2) as reporter antibody gave a 2-fold increase in counts detected (10,810 CPM) compared to the "original" configuration of antibody presentation (5,825). Thus, these results are consistent with the data obtained in Table 7 using the sera from 18 cancer patients with the "reverse" configuration of antibody presentation.

4. The "reverse" configuration of v-H-ras (Ab-1) as the capture matrix and v-H-ras (Ab-2) as the reporter produced 20-fold higher counts (10,810) than using the v-H-ras (Ab-1) pantropic antisera for both matrix and reporter (506 CPM). The "original" configuration of v-H-ras (Ab-2) as the capture matrix and v-H-ras (Ab-1) as the reporter yielded a five-fold increase in counts obtained (5,825 CPM) compared to v-H-ras (Ab-2) used for both matrix and reporter (1,660 CPM). Thus, not only is the background of non-specific antibody binding lower, using v-H-ras (Ab-1) as the capture matrix, but the sensitivity of the system is also greatly increased compared to utilizing v-H-ras (Ab-2) as the capture matrix (first step). Further experimentation is necessary before one can determine whether utilizing a pantropic or polyclonal antisera as the first step, capture matrix, is a superior and more sensitive approach for the antigen capture procedure.

5. Non-specific activity was detected using v-H-ras (Ab-2) as the reporter antibody with the control capture matrices v-fms (Ab-2) (2,821; 209) and cytokeratin (Ab-1) (2,073). The v-H-ras (Ab-1) did not react with the v-fms (Ab-2) capture matrix (359), therefore, providing another example for its possible superiority as a reporter antibody as well as a capture matrix antibody.

6. Both control capture matrices containing v-fms (Ab-2) and cytokeratin (Ab-1) antibodies showed no reactivity with normal human sera using either of the anti-ras monoclonal antibodies as reporters; however, a low number of counts (209) was obtained with the anti-fms (Ab-2) as reporter. Also, a low number of counts was observed using the control cytokeratin (Ab-1) as capture matrix and both anti-ras antibodies as reporter (1,106; 2,073). If the sera contained cytokeratin protein, which was retained by the cytokeratin (Ab-1) capture matrix, the reporter antibodies specific for ras protein would not bind to the cytokeratin antigen, therefore the observation of counts above background in both of these instances is probably due to non-specific binding inherent to the system.

X. Antigen Capture Procedure Using the Sera of Apparently Normal Humans.

In order to substantiate the use of the antigen capture procedure as a positive test for the specific detection of ras gene encoded protein in the sera of patients with various malignancies, the test should be successful in obtaining negative results with a large number of sera from normal human donors. Forty-six normal human sera samples were incubated with both or either configuration of antibody presentation, i.e., v-H-ras (Ab-2) capture matrix with v-H-ras (Ab-1) reporter antibody and v-H-ras (Ab-1) capture matrix with v-H-ras (Ab-2) reporter antibody. As a positive control some of the normal human sera samples were compared to the same volume of sample (0.5 ml) containing 0.05 ml of cellular extract from Al-1 cells (7.0 mg/ml total protein; lot number A106). Two of the forty-six normal sera samples demonstrated high results (patient number 5303-5 and 5303-13) using the v-H-ras (Ab-2) as the capture matrix . The average of the other samples was approximately 300 CPM using this capture matrix. As expected six of these normal human sera which had equal amounts of ras p21 containing extract added, showed a 4-7 fold increase in counts compared to normal counterparts.

XI. Quantitation of ras p21 by Solution-Phase Antigen Capture Procedure.

In an attempt to improve the specificity, sensitivity and reproducibility of ras p21 detection, a solution phase antigen capture test was evaluated. This assay was performed as described in "Materials and Methods." As shown in Figure 2, the standard curve of the solution-phase antigen capture test was linear with respect to ras p21 for values between 1 and 50 units of p21 in which 1 unit is approximately equal to 1 ng of purified ras p21. Internal standards A, B, C containing 23.75, 6.25 and 1.25 units ras p21 reactivity respectively were assayed with each standard curve and were found to fall on the curve at all times. The reproducibility of the internal standards in the assay demonstrates the very low interassay variability of the second generation antigen capture test. Although the data shown was obtained by assaying frozen aliquots of p21 antigen similar results were obtained by assay of lyophilized and reconstituted standards and internal standards. Stability studies conducted on the standards and other assay components showed no deterioration of components for the 60 days shelf life of the $^{125}$Iodine labeled v-H-ras (Ab-2) reporter antibody.

XII.   Solution-Phase Antigen Capture Analysis of ras
       p21 in Cell Line Extracts.

In order to further evaluate the solution phase antigen capture assay, levels of ras encoded p21 in extracts of cell lines were assayed. As shown in Table 9, the Al-1 cell line contains 1000 units of ras p21 reactivity per mg of total cellular extract protein, and this reactivity corresponds to the human c-H-ras gene encoded protein.   The OS-8 cell line known to be transfected with the human c-K-ras gene was found to contain 725 units of ras p21 reactivity per mg total cellular extract protein; and the HL-60-2 cell line, expressing human c-N-ras p21, assay result was 102 units per mg cellular extract protein.   The c-H-ras p21 levels in the Al-1 cell line represent an arbitrary estimate based on the results of the purification experiments, and one unit is approximately equal to one nanogram of c-H-ras p21. The relative levels of $^{35}$S-Methionine labeled p21 immunoprecipitable from these cell lines was estimated to be equal for Al-1 and OS-8 and approximately 50 percent lower for HL-60-2 (data not shown).   These data indicate that the solution phase antigen capture test measures c-H-ras and K-ras encoded p21 with equal sensitivity but is slightly less sensitive for c-N-ras p21.

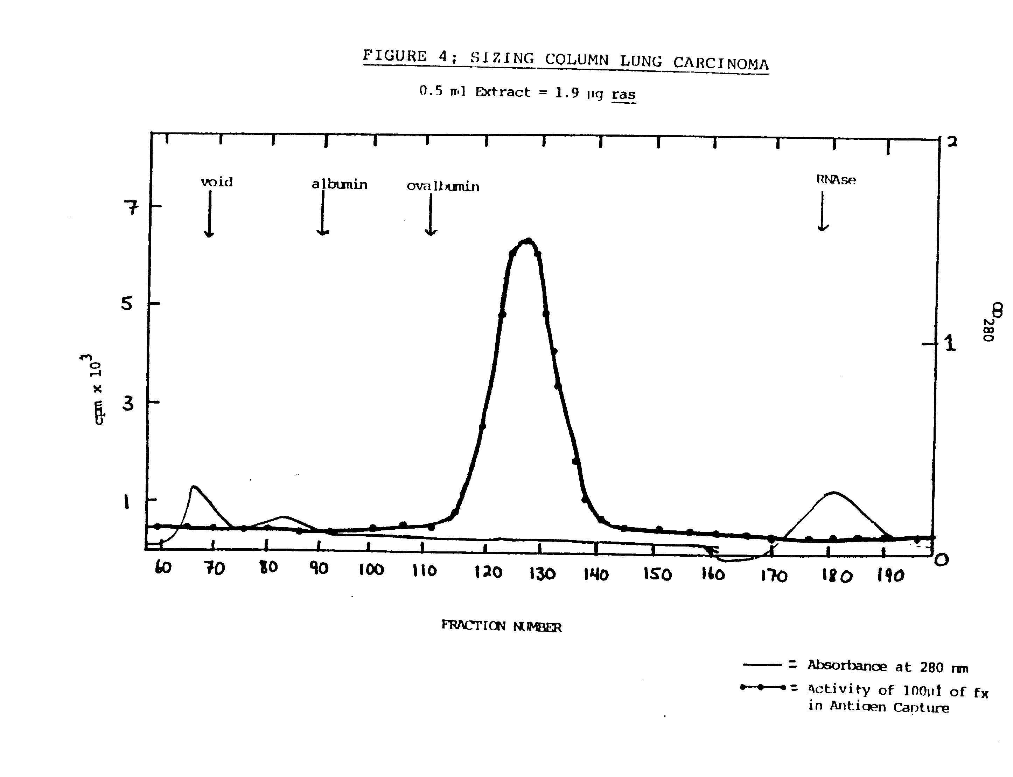

The Al-1 cellular extract was subjected to gel filtration over a column of AcA 54 (LKB Rockville, MD), that had been previously calibrated with molecular weight markers, and the fractions were analyzed for ras p21 content by antigen capture and Western blotting, Figure 3.   Figure 4 shows similar data for a representative lung carcinoma.   In both cases the p21 antigen capture

TABLE  9  :  <u>LEVELS OF <u>ras</u> p21 IN EXTRACTS OF HUMAN</u>

<u>ras</u>  <u>TRANSFECTED CELL LINES</u>

| Cell[a] Line | Description | Mg Protein Assayed | cpm[b] | Units[c] p21/Mg Total Protein |
|---|---|---|---|---|
| A1-1 | Mouse cells transfected with mutated Human c-H-<u>ras</u> gene | .050 | 28906 | 1000 |
| OS-8 | Mouse cells transfected with mutated Human c-K-<u>ras</u> gene | .050 | 13179 | 725 |
| HL-60-2 | Mouse cells transfected with mutated Human c-N-<u>ras</u> gene | .160 | 5623 | 102 |

Table 9: Continued

<sup>a</sup> Cellular extracts were prepared as described under Materials and Methods.

<sup>b</sup> Extract (0.1 ml), 3 µg biotinylated capture antibody and 100,000 cpm of $^{125}$iodine-labeled reporter antibody were combined, incubated one hour at 25°C, and 16 µg strepavidin covalently coupled to agarose were added. Following 30 minutes of incubation at 25° with shaking, the immunocomplexes were collected by centrifugation at 2,800 rpm for 3 minutes in a Beckman® refrigerated centrifuge (model TJ-6), supernatant containing unbound reporter antibody was aspirated and 3 ml of PBS 0.1% Triton® X-100 was added. The centrifugation, aspiration and wash steps were repeated three times, and the bound $^{125}$Iodine counts were measured in a LKB gamma counter (Model 1274).

<sup>c</sup> One unit of <u>ras</u> p21 is approximately equal to one ng p21.

0214520

- 67 -

reactivity and Western blotting reactivity eluted from the column at a position corresponding to a molecular weight of 21,000.

XIII. Solution-Phase Antigen Capture Procedure Using Sera from Cancer Patients and Apparently Normal Humans.

To establish the validity of measuring p21 levels in cancer patient sera as a means of cancer detection or monitoring, the solution-phase antigen capture test was used in a series of control experiments and for the testing of both cancer patient and control human sera. In one control experiment the ras p21 content of cancer and normal human sera were compared and biotinylated capture antibody (v-H-ras Ab-1) was replaced by anti-p53 and v-fms monoclonal antibodies (Table 10). Testing of a cancer patient's sera (0.1 ml) resulted in a value of 1815 cpm, over a background value of 174 cpm for normal sera, and this corresponds to 65 units ras p21 per ml patient sera. The substitution of anti-p53 as the capture antibody drastically reduced the bound $^{125}$Iodine-labeled reporter antibody to 119 cpm over background; a similar value of 59 cpm was obtained by the use of anti v-fms antibody. In another series of control experiments the measurement of ras p21 in cancer patient sera was found to increase linearly over a sera volume range of 0.05 to 0.2 ml.

Sera from apparently healthy humans was assayed in this test and as shown on Figure 5, the levels of p21 were found to be below 3 units of ras p21 for the vast majority of samples obtained from either of two sources Interstate Blood Bank (P.O. Box 393, Memphis, Tennessee 38101) and Clinical Concepts (689 Front Street,

TABLE 10: <u>ANTIGEN CAPTURE PROCEDURE VARYING BIOTINYLATED ANTIBODIES USED FOR CAPTURE</u>

| Sera Sample | Capture Antibody | Reporter Antibody | Average CPM | CPM Minus Background |
|---|---|---|---|---|
| Colon cancer | v-H-<u>ras</u> Ab-1 | v-H-<u>ras</u> Ab-2 | 1989 | 1815 |
| Normal | v-H-<u>ras</u> Ab-1 | v-H-<u>ras</u> Ab-2 | 174 | - |
| Colon cancer | p53 Ab-1 | v-H-<u>ras</u> Ab-2 | 289 | 119 |
| Normal | p53 Ab-1 | v-H-<u>ras</u> Ab-2 | 170 | - |
| Colon cancer | v-<u>fms</u> Ab-1 | v-H-<u>ras</u> Ab-2 | 347 | 59 |
| Normal | v-<u>fms</u> Ab-1 | v-H-<u>ras</u> Ab-2 | 288 | - |

a. Sera sample (0.1 ml), 3 ug biotinylated capture antibody and 100,000 cpm of $^{125}$iodine labeled reporter antibody were combined, incubated one hour at $25^{\circ}$C, and 16 ug streptavidin covalently coupled to agarose were added. Following, 30 min. of incubation at $25^{\circ}$ with shaking, the immunocomplexes were collected by centrifugation at 2,800 rpm for 3 min. in a Beckman refrigerated centrifuge (Model TJ-6), supernatant containing unbound reporter antibody was aspirated and 3 ml of PBS 0.1% Triton® X-100 was added. The centrifugation, aspiration and wash steps were repeated three times, and the bound $^{125}$Iodine counts were measured in LKB gamma counter (Model 1274).

Teaneck, New Jersey 07666). There were, however, a few sera which assayed positively for p21 in both sets of samples. These values did not change on repeated assay of the same samples. The majority of the sera from cancer patients tested positive for ras p21 (Figure 6). Although the two bladder cancer sera tested negative, earlier sera data for bladder cancer patients were positive suggesting that the results may reflect the success or failure of therapy. The colon cancer patient sera tested contained 14 values above the mean of normal sera controls and 7 below the mean. A similar distribution was obtained for ovarian cancer patient sera with 13 positives out of 22 samples. The melanoma cancer patient sera all tested positive, but all of these patients were known to have advanced cancer. The levels of p21 were found to correlate with the relative success or failure of therapy.

Advanced cancer patients, receiving chemotherapy, were monitored for serum ras p21 levels at multiple times before, during and following therapy. A melanoma patient who responded to chemotherapy had an initial serum p21 level of 45 ng/ml which dropped to 17 ng/ml a week after chemotherapy. Within 2 months the patient relapsed and the p21 level returned to 40 ng/ml. Three other melanoma patients studied failed to respond to therapy, and their serum p21 levels either remained high or increased as the disease progressed. An advanced bladder cancer patient who failed to respond to surgery, radiation therapy and chemotherapy exhibited increasing serum p21 levels until he expired. Ovarian cancer patients failing to respond to surgery, chemotherapy and radiation therapy showed increasing serum p21 levels. Patients responding to therapy showed decreasing serum p21 levels. Similar trends were noted in other cancer patients that were followed for serum p21 levels suggesting that this test will be useful to monitor cancer therapy.

The antigen capture reactivity from serum was purified by monoclonal affinity chromatography and was found to Western blot at 21,000 daltons (Fig. 7). The purified ras reactivity was subjected to molecular sizing over a column of AcA 54 and chromatographed between the void volume of the column and the BSA marker. In data not shown, the ras p21 reactivity of cancer patient sera was found to Western blot at 21,000 daltons. These results indicate that the ras p21 found in human serum is contained in noncovalent complex of approximately 100,000 daltons.

DISCUSSION

Activation of members of the ras oncogene family by point mutations at specific positions (i.e., codons 12, 13 & 61) is known to be associated with broad range of human tumors. Thus of all of the human oncogenes studied to date, the ras system holds the greatest promise as the basis of a test for the early detection, differential diagnosis and monitoring of human cancer. The major emphasis of attempts to develop such a test to date have focused on nucleic acid sequencing and more recently oligonucleotide differential melt Southern hybridization procedures to directly identify mutated ras oncogenes. An alternative approach, explored to a lesser extent to date, is the use of ras p21 specific monoclonal antibodies to measure elevated ras oncogene expression by aminohistopathology. These procedures have the common problem of requiring tumor biopsy material and thus are not readily amenable to either early cancer detection or monitoring of tumor progression.

Although ras p21 is a cellular membrane protein and thus not secreted physiologically into the blood stream, its presence in serum at appreciable levels is possible if there is sufficient tumor cell destruction. A determination as to whether ras p21 is present in human serum is important with respect to the wider implications of such a determination to other oncogene systems. The demonstration of detectable levels of a representation of non-secreted oncogene encoded protein, such as ras p21, in human serum, shows that the phenomenon may be more general and that serum-based immunoassays may have wider application encompassing a broad range of oncogene systems and oncogene-encoded proteins.

The initial approach to the development of an immuno-logic test for ras p21 was to select a system that was highly sensitive, but also adaptable to a wide range of formats and applications. The antigen capture procedure was chosen for several reasons. The approach involves the use of a broadly reactive antibody coupled to a solid support matrix to purify and concentrate the oncogene-encoded protein, in this case ras p21, from cellular extracts, serum, or other biological material. The second antibody, coupled to an indicator, either isotopic or non-isotopic, is then used to demonstrate the bound antigen. This procedure is adaptable to a wide range of manual and automated formats for antigen concentration by the first antibody. In addition the use of the coupled second antibody as the detector systems makes possible the adaptation of the test to any of a wide range of available colormetric, fluorescent, luminescent or isotopic systems. The use of the coupled second antibody as a detector allows format-

ting of the test to make specific determinations as to the nature of the bound antigen. For instance, in the ras p21 oncogene system, it is possible to select detector reagents that discriminate H-, N- and K-ras, and even that recognize as to whether any one of the ras oncogene products has a point mutation at one of the positions known to be critical to ras oncogene activation. Similarly, the same format could be applied to other oncogene systems by selecting detector antibodies that discriminate the activated from the non-activated form of the oncogene-encoded protein.

Using the above described assay with a monoclonal antibody specific for one epitope of ras p21 bound to agarose beads as the capture system and a monoclonal antibody to an independent epitope of ras p21 coupled with $^{125}$I as the detector, we were able to detect ras p21 protein in sera. The specificity of the assay for ras p21 was demonstrated by analysis of extracts of a series of rodent and human cell lines with well defined levels of ras p21 expression. In contrast, when a monoclonal antibody against cytokeratin 18 was used as a control, either for the capture or detector side of the test, little or no detectable reactivity was observed.

On the basis of the present results, it is clear that serum levels of ras p21 differ among individual patients and that in many cases the levels are not significantly higher than in sera of non-cancer patients. However, the correlation of the detection of ras p21 in putative cancer patients but not in the sera from a large number of normal donors strengthens the specificity and reliability of the test as a diagnostic aid specific for cancer.

Information derived from these studies also showed that enhanced sensitivity of the assay can be achieved by varying the monoclonal antibody used in the first step for capture or in the second step as detector-reporter. Thus, manipulation of the test system by using combinations of monoclonal and/or polyclonal antibodies of different determinant specificities, affinities or avidities in a varied order of presentation could increase the number of cancer patients that ras-encoded protein may be detected. It is possible that those cancer patients with the lower ras p21 levels may be in remission, or alternatively that their cancers may have been induced by genetic mechanisms independent of the ras oncogenes system. To resolved these questions, serial evaluation of ras p21 levels in sera from cancer patients with well-defined clinical histories are necessary. At the same time, tumor tissue samples will have to be obtained from patients and their DNAs analyzed directly for single base change mutations at those positions in the ras oncogene (i.e., codons 12, 13 and 61) involved in their activation. By this approach it will also be possible to make a determination as to whether the form of ras p21 detected in the serum of human cancer patients correlates with the member of the ras oncogene family that is mutated from an inactive to active form. These findings constitute the first demonstration of elevated levels of an oncogene-encoded protein in serum of human cancer patients and provide a sensitive and specific immunologic assay format for its detection.

REFERENCES

1.  Anderson, S.J., Furth, M. Wolff, L., Ruscetti, S.K. and Sherr, C.J. (1982). Monoclonal antibodies to the transformation-specific glycoprotein encoded by the feline retroviral oncogene v-fms. J. Virol. 44, 696-702.

2.  Bishop, J.M. (1981). in: Developmental Biology Using Purified Genes, ed. Brown, (Academic, New York.) pp. 514-525.

3.  Bishop, J.M. (1982). Retroviruses and cancer genes. Adv. in Cancer Res. 37, 1-69.

4.  Bishop, J.M. (1982). Oncogenes. Sci. Amer. 246, 89-92.

5.  Bonner, W.M. and Laskey, R.A. (1974). Detection method for Tritium-labeled proteins and nucleic acids in polyacrylamide gels, Eur. J. Biochem 46, 83-88.

6.  Cooper, G. (1982). Cellular transforming genes. Science 217, 801-806.

7.  Debus, E., Weber, K. and Osborn, M. (1982). Monoclonal cytokeratin antibodies that distinguish simple from stratified epithelia: Characterization on human tissues. EMBO Journal 12, 1641-1647.

8.  Der, C., Krontiris, T., and Cooper, G. (1982). Transforming genes of human bladder and lung carcinoma cell lines are homologous to the ras genes of Harvey and Kirsten sarcoma viruses. Proc. Nat. Acad. Sci. 79, 3637-3640.

9. Der, C.J. and Cooper, G.M. (1983). Altered gene products are associated with activation of cellular ras<sup>k</sup> genes in human lung and colon carcinomas. Cell 32, 201-208.

10. Ellis, R.W., Defeo, D., Shih, T.Y., Gonda, M.A., Young, H.A., Tsuchida, N., Lowry, D.R., and Scolnick, E.W. (1981). The p21 src gene of Harvey and Kirsten sarcoma viruses originate form divergent members of a family of normal vertebrate genes. Nature 292, 506-511.

11. Furth, M.E., Davis, L.J., Fleurdelys, B., and Scolnick, E.M. (1982). Monoclonal antibodies to the p21 products of the transforming gene of Harvey murine sarcoma virus and of the cellular ras gene family. J. Virol. 43, 294-304.

12. Gallick, G.E., Kurzrock, R., Kloetzer, W.S., Arlinghaus, R.B., and Gutterman, J.V. (1985). Expression of p21 ras in fresh primary and metastatic human colorectal tumors. Proc. Natl. Acad., Sci. 82, 1795-1799.

13. Hand, P.H., Thor, A., Wunderlich, R., Muraro, A., Carusso, A., and Schlom, J. (1984). Monoclonal antibodies of predefined specificity detect activated ras gene expression in human mammary and colon carcinoas. Proc. Natl. Acad. Sci. 81, 5227-5231.

14. Harlow, E., Crawford, L.V., Pim, D.C. and Williamson, N.M. (1981). Monoclonal antibodies specific for simian virus 40 tumor antigens. J. Virol. 39, 861-869.

15. Hayward, W.S., Noel, B.G., and Astrin S.M. (1981). Activation of a cellular onc gene by promoter insertion in ALV - induced lymphoid leukosis. Nature 290, 475-480.

16. Huebner, R.J. and Todaro, G. (1969). Oncogenes of RNA tumor viruses as determinants of cancer. Proc. Nat. Acad. Sci. 64, 1087-1094.

17. Hurley, J.B., Simon, M.I., Teplow, D.B., Robishaw, J.D., and Gilman, A.G. (1985). Homologies between signal transducing G proteins and ras gene products. Science 226, 860-862.

18. Klein, G. ed. (1980). in: Advances in Viral Oncology, vol. 1. (Raven press, New York) pp. 1-262.

19. Laemmli, U.K. (1970). Cleavage of structural proteins during assembly of the head of bacteriophage T4. Nature 227, 680-685.

20. Land, H., Parada, L.F. and Weinberg, R.A. (1983). Cellular oncogenes and multistep carcinogenesis. Science 222, 771-778.

21. Lowry, O.H., Rosebrough, N.J., Farr, A.L., and Randall, R.J. (1951). Protein measurement with the folin phenol reagent. J. Biol. Chem. 193, 265-275.

22. McGrath, J.P., Capon, D.J., Goeddel, D.V. and Levinson, A.D. (1984). Comparative biochemical properties of normal and activated human ras p21 protein. Nature 310, 644-649.

23. Merril, C.R., Goldman, D., Sedman, D., and Ebert, M.H. (1981). Ultrasensitive stain for proteins in polyacrylamide gels shows regional variation in cerebrospinal fluid proteins. Science 211, 1437-1438.

24. Shimizu, K., Birnbaum, D., Ruley, M., Fasano, O., Suard, Y., Edlund, L., Taparowsky, E., Goldfarb, M., and Wigler, M. (1983). The structure of the k-ras gene of the human lung carcinoma cells line Calu-1. Nature 304, 497-500.

25. Slamon, D.J., de Kerniou, J.B., Verma, I.M. and Cline, M.J. (1985). Expression of cellular oncogenes in human malignancies. Science 224, 256-262.

26. Spector, D.H., Varmus, H.E., and Bishop, J.M. (1978). Nucleotide sequences related to the transforming gene of avian sarcoma virus are present DNA of uninfected vertebrates. Proc. Nat. Acad. Sci. 75, 4102-4106.

27. Tabin, C., Bradley, S., Bargmann, C., Weinberg, R., Papageorge, A., Scolnick, E., Dhar, R., Lowy, D., and Chang, E. (1982). Mechanism of activation of a human oncogene. Nature 300, 143-148.

28. Taparowsky, E., Suard, Y., Fasano, O., Shimizu, K., Goldfarb, M., Wigler, M. (1982). Activation of the T24 bladder carcinoma transforming gene is linked to a single amino acid change. Nature 300, 762-765.

29. Taparowsky, E., Shimizu, K., Goldfarb M., and Wigler M. (1983). Structure and activation of the human N-ras gene. Cell 34, 581-586.

30. Van de Ven, W.M., Reynolds, F.H. Jr., and Stephenson, J.R. (1980). The nonstructural components of polyproteins encoded by replication - defective mammalian transforming. Retroviruses are phosphorylated and have associated protein kinase activity. J. Virol. 101

31. Veronese, F., Kelloff, G.J., Reynolds, Jr., F.H.,- Hill, R.W., and Stephenson, J.R. (1982). Monoclonal antibodies specific to transforming polyproteins encoded by independent isolates of feline sarcoma virus. J. Virol. 43, 896-904.

32. Weiss, R., Teich, N., Varmus, H. and Coffin J. (1984). RNA Tumor Viruses: The Molecular Biology of Tumor Viruses part III. (Cold Spring Harbor Monograph, 1/Text, New York).

What is Claimed is:

1.  A method for diagnosing in a subject a neoplastic condition associated with the presence of an activated oncogene which comprises detecting in a sample of a biological fluid from the subject the presence of at least a portion of a polypeptide encoded by the activated oncogene, the polypeptide normally occurring intracellularly.

2.  A method of claim 1, wherein the subject is human.

3.  A method of claim 1, wherein the subject is an animal.

4.  A method of claim 1, wherein the activated oncogene is c-H-ras.

5.  A method of claim 1, wherein the activated oncogene is c-K-ras.

6.  A method of claim 1, wherein the activated oncogene is c-N-ras.

7.  A method of claim 1, wherein the activated oncogene is c-myc.

8.  A method of claim 1, wherein the activated oncogene is c-N-myc.

9.  A method of claim 1, wherein the activated oncogene is c-L-myc.

10. A method of claim 1, wherein the activated oncogene is c-abl.

11. A method of claim 1, wherein the activated oncogene is c-fos.

12. A method of claim 1, wherein the activated oncogene is c-R-myc.

13. A method of claim 1, wherein the activated oncogene encodes the polypeptide p53.

14. A method of claim 1, wherein the biological fluid is sera.

15. A method of claim 1, wherein the biological fluid is urine.

16. A method of claim 1, wherein the biological fluid is cerebro-spinal fluid.

17. A method of claim 1, wherein the biological fluid is amniotic fluid.

18. A method of claim 1, wherein the biological fluid is sputum.

19. A method of claim 1, wherein the biological fluid is a lung lavage.

20. A method of claim 1, wherein the biological fluid is ascites fluid.

21. A method of claim 1, wherein the biological fluid is saliva.

22. A method of claim 1, wherein the biological fluid is a mucous-type bodily secretion.

23. A method of claim 1, wherein the biological fluid is blood.

24. A method of claim 1, wherein the biological fluid is plasma.

25. A method of claim 4, wherein the polypeptide encoded by the activated oncogene is c-H-ras p21.

26. A method of claim 6, wherein the polypeptide encoded by the activated oncogene is c-N-ras p21.

27. A method of claim 5, wherein the polypeptide encoded by the activated oncogene is c-K-ras p21.

28. A method of claim 7, wherein the polypeptide encoded by the activated oncogene is c-myc p62 and p65.

29. A method of claim 8, wherein the polypeptide encoded by the activated oncogene is c-N-myc p64 and p66.

30. A method of claim 12, wherein the polypeptide encoded by the activated oncogene is the c-R-myc encoded polypeptide.

31. A method of claim 9, wherein the polypeptide encoded by the activated oncogene is c-L-myc encoded polypeptide.

32. A method of claim 11, wherein the polypeptide encoded by the activated oncogene is c-fos p55.

33. A method of claim 1, wherein the polypeptide encoded by the activated oncogene is p53.

34. A method of claim 1, wherein the polypeptide encoded by the activated oncogene is a fusion polypeptide in part encoded by the oncogene and in part by the chromosomal DNA of the subject.

35. A method of claim 34, wherein the polypeptide encoded by the activated oncogene is phl/c-abl p210.

36. A method of claim 1, wherein the polypeptide encoded by the activated oncogene is a polypeptide normally associated with the inner cell membrane.

37. A method of claim 1, wherein the polypeptide encoded by the activated oncogene is a polypeptide normally associated with the nucleus.

38. A method of claim 1, wherein the detecting comprises contacting the sample under suitable conditions with a first matrix-bound antibody specific for the portion of the polypeptide to form a matrix first antibody polypeptide complex, contacting the complex so formed with a second antibody labelled with a detectable marker to form a second complex comprising matrix-first antibody-polypeptide-second antibody and detecting the second complex so formed, thereby detecting the presence of the oncogene-encoded polypeptide.

39. A method of claim 38, wherein the second antibody is a monoclonal antibody.

40. A method of claim 38, wherein the second antibody is a polyclonal antibody.

41. A method of claim 38, wherein the second antibody is bound to a matrix.

42. A method of claim 41, wherein the matrix is agarose.

43. A method of claim 41, wherein the matrix is Sepharose®.

44. A method of claim 38, wherein the first antibody is bound to a tube.

45. A method of claim 38, wherein the first antibody is bound to a bead.

46. A method of claim 38, wherein the second antibody is labelled with a detectable marker.

47. A method of claim 46, wherein the detectable marker is a radioactive label.

48. A method of claim 47, wherein the radioactive label is $^{125}I$.

49. A method of claim 46, wherein the detectable marker is a colorometric marker.

50. A method of claim 46, wherein the detectable marker is a fluorometric marker.

51. A method of claim 46, wherein the detectable marker is a luminescent marker.

52. A method of claim 46, wherein the detectable marker is the product of an enzymatic reaction.

53. A method of claim 1, wherein the detecting comprises contacting the sample under suitable conditions with an antibody specific for the portion of the polypeptide to be detected to form an antibody-polypeptide complex, detecting the complex so formed and thereby detecting the presence of the oncogene-encoded polypeptide.

54. A method of claim 53, wherein the antibody is a monoclonal antibody.

55. A method of claim 53, wherein the antibody is a polyclonal antibody.

56. A method of claim 53, wherein the antibody is one of a selected combination of antibodies.

57. A method of claim 53, wherein the antibody is labelled with a detectable marker.

58. A method of claim 57, wherein the detectable marker is a radioactive label.

59. A method of claim 58, wherein the radioactive label is $^{125}I$.

60. A method of claim 57, wherein the detectable marker is a colorometric marker.

61. A method of claim 57, wherein the detectable marker is a fluorometric marker.

62. A method of claim 57, wherein the detectable marker is a luminescent marker.

63. A method of claim 57, wherein the detectable marker is the product of an enzymatic reaction.

64. A method of claim 53, wherein the complex forms a detectable immunoprecipitate.

65. A method of claim 1, wherein the detecting comprises contacting a detectable control polypeptide with an antibody specific for the portion of the polypeptide in the presence of the oncogene-encoded polypeptide, thereby forming an antibody-control polypeptide complex, quantitatively determining the number of complexes so formed and comparing the number so determined with the number of complexes formed in the absence of the oncogene-encoded polypeptide, a decrease in the number of complexes formed indicating the presence of the oncogene-encoded polypeptide in the sample.

66. A method of claim 65, wherein the antibody is a monoclonal antibody.

67. A method of claim 65, wherein the antibody is a polyclonal antibody.

68. A method of claim 65, wherein the antibody is one of a selected combination of antibodies.

69. A method of claim 65, wherein the antibody is bound to a matrix.

70. A method of claim 69, wherein the matrix is agarose.

71. A method of claim 69, wherein the matrix is Sepharose®.

72. A method of claim 69, wherein the antibody-control polypeptide complex is separated from solution by a second antibody combining with the antibody of the antibody-control polypeptide complex.

73. A method of claim 65, wherein the antibody is bound to a tube.

74. A method of claim 65, wherein the antibody is bound to a bead.

75. A method of claim 65, wherein the control polypeptide is labelled with a detectable marker.

76. A method of claim 75, wherein the detectable marker is a radioactive label.

77. A method of claim 76, wherein the radioactive label is $^{125}$I.

78. A method of claim 75, wherein the detectable marker is a colorometric marker.

79. A method of claim 75, wherein the detectable marker is a fluorometric marker.

80. A method of claim 75, wherein the detectable marker is a luminescent marker.

81. A method of claim 75, wherein the detectable marker is the product of an enzymatic reaction.

82. A method of claim 1, wherein the detecting comprises contacting the sample under suitable conditions with a first antibody specific for an epitope on the portion of the polypeptide to be detected, and with a second antibody specific for a different epitope on the portion of the polypeptide to be detected so as to form a first antibody-polypeptide-second antibody complex, the complex being detectable.

83. A method of claim 82, wherein the first antibody is a monoclonal antibody.

84. A method of claim 82, wherein the first antibody is a polyclonal antibody.

85. A method of claim 82, wherein the second antibody is a monoclonal antibody.

86. A method of claim 82, wherein the second antibody is a polyclonal antibody.

87. A method of claim 82, wherein detection is effected by means of an enzymatic reaction.

88. A method of claim 82, wherein detection is effected by means of a chemical reaction.

89. A method of claim 82, wherein detection is effected by means of a wavelength shift of absorbed length.

90. A method of claim 82, wherein detection is effected by means of emission of light.

91. A method of claim 82, wherein detection is effected by means of radioactivity.

92. A method for diagnosing in a subject a neoplastic condition associated with the presence of an activated oncogene which comprises quantitatively determining in a sample of a biological fluid from the subject the amount of an oncogene-encoded polypeptide and comparing the amount of the polypeptide so determined to the amount in a sample from a normal subject, the presence of a significantly different amount indicating the presence of the neoplastic condition.

93. A method for monitoring the course of a neoplastic condition in a subject which comprises quantitatively determining in a first sample of a biological fluid from the subject the presence of an oncogene-encoded polypeptide and comparing the amount so determined with the amount present in a second sample from the subject, such samples being taken at different points in time, a difference in the amounts determined being indicatives of the course of the neoplastic condition.

94. A method for typing tumors which comprises detecting in a sample of a biological fluid from a subject with a neoplastic condition the presence of one or more oncogene-encoded polypeptides in the sample, the presence or absence of a specific combination thereof being indicative of a specific tumor type.

95. A method for typing tumors which comprises quantitatively determining in a sample of a biological fluid from a subject with a neoplastic condition the amount of one or more oncogene-encoded polypeptides in the sample, the presence of specific amounts or relative amounts thereof being indicative of a specific tumor type.

96. A method for screening putative therapeutic agents for the treatment of a neoplastic condition which comprises quantitatively determining in a first sample from a subject with the neoplastic condition the amount of an oncogene-encoded polypeptide associated with the condition, administering to the subject a therapeutic amount of the agent such that the agent is contacted with a neoplastic cell associated with the condition to produce a treated subject, determining after a suitable period the amount of the polypeptide in a sample from the treated subject, and comparing the amount of polypeptide determined in the first sample with the amount determined in the sample from the treated subject, a significant difference indicating the effectiveness of the agent.

97. A method for detecting an activated oncogene which comprises detecting in a sample of a biological fluid at least a portion of a polypeptide encoded by the activated oncogene, the polypeptide occurring intracellularly.

98. A method of claim 38, wherein the first matrix-bound antibody is produced by hybridoma cell line designated ATCC No. CRL1742.

99. A method of claim 38 wherein the second antibody is produced by hybridoma cell line designated ATCC No. CRL1741.

100. A method of claim 53, wherein the antibody is produced by hybridoma cell line designated ATCC No. CRL1742.

101. A method of claim 65, wherein the antibody is produced by hybridoma cell line designated ATCC No. CRL1742.

102. A method of claim 72, wherein the second antibody is produced by hybridoma cell line designated ATCC No. CRL1741.

103. A method of claim 82, wherein the first antibody is produced by hybridoma cell line designated ATCC No. CRL1742.

104. A method of claim 82, wherein the antibody is produced by hybridoma cell line designated ATCC No. CRL1741.

FIGURE 1: KINETICS OF SOLID PHASE ANTIGEN
CAPTURE REACTION

Antigen Capture
A1-1 binding to:
● Capture Matrix v-H-ras(Ab-2)
○ Capture Matrix cytokeratin(Ab-1)
△ Delta

FIGURE 2: STANDARD CURVE OF RAS p21 SOLUTION-PHASE
ANTIGEN CAPTURE ASSAY

# FIGURE 3: SIZING COLUMN A1-1 CELL EXTRACT

0.25 ml ( 24 mg Protein) = 780$_{ng}$ ras

FRACTION NUMBER

●—● = activity of 100µl of fraction in Antigen Capture

FIGURE 4; SIZING COLUMN LUNG CARCINOMA

0.5 ml Extract = 1.9 µg ras

Legend:
—— = Absorbance at 280 nm
•—•—• = Activity of 100µl of fx in Antigen Capture

FIGURE 5: RAS p21 REACTIVITY IN CONTROL SERA

FIGURE 6: RAS p21 REACTIVITY IN CANCER
PATIENT SERA

FIGURE 7: ras RECOVERED FROM NORMAL SERA ON IMMUNOAFFINITY COLUMN

7/7 021452